# EUROPEAN PATENT APPLICATION

(11) **EP 4 538 694 A1**
(43) Date of publication of application: **16.04.2025**
(21) Application number: 24206278.4
(22) Date of filing: 11.10.2024
(51) Int. Cl.: G01N 29/032, G01N 29/22

(54) **ULTRASONIC MICROPLASTIC DETECTION SENSOR APPARATUS AND METHOD**

(30) Priority: 12.10.2023 US 202363543758 P; 19.07.2024 US 202418778841
(71) Applicant: Applied Ocean Sciences LLC, Springfield, VA 22151 (US)
(72) Inventor: Verlinden, Christopher Michael Alexander, Clarksburg, MD 20871 (US); Brandon, Jennifer, Encinitas, CA 92024 (US); Sevick, John, Fredericksburg, VA 22405 (US); Murray, James, Stafford, VA 22554 (US); Seger, Kerri, Seattle, WA 98107 (US)
(74) Representative: FRKelly

(57) **Abstract**

An apparatus configured to detect small particles of a substance suspended in a matrix using ultrasound, may include: a container device configured to receive a sample in an inner volume, including: a first ultrasonic transducer mechanically coupled to or disposed on a first exterior portion of the container device and oriented toward the inner volume, the first ultrasonic transducer may include: a first ultrasonic transmitter; and a first ultrasonic receiver; optionally a first receiver amplifier coupled to the first receiver; optionally the first transmitter amplifier coupled to the first transmitter amplifier; and either: a) a software defined radio (SDR), or b) a signal generator and an oscilloscope, where SDR or signal generator and oscilloscope are electronically coupled to the first ultrasonic transmitter and electronically coupled to the first ultrasonic receiver amplifier; and the electronic controller coupled to the SDR or the signal generator and the oscilloscope.

## Description

### CROSS-REFERENCE TO RELATED APPLICATIONS

None

### GOVERNMENT CONTRACT

This disclosure was prepared related to research performed by Applicant under an SBIR NSF Contract 2136729, awarded December 8, 2021.

### BACKGROUND OF THE DISCLOSURE

### FIELD OF THE DISCLOSURE

The disclosure is related generally to detection apparatuses and more particularly to apparatuses for detecting particulates in an aqueous solutions.

### Related Art of the Disclosure

Pollution of the Earth's water supplies is a widely known global challenge.

Scientists have long believed that plastics may never fully biodegrade, and may only break down over time into smaller and smaller pieces.

With widespread use of plastic containers in the late 20^{th} and early 21^{st} centuries, plastics pollution of water has reached critical levels. As materials like plastics break down, the materials may become of continually smaller size making difficult the task to detect such minute particles in aqueous and/or matrix samples.

Generally, tiny plastic particles of 5 millimeters or smaller, are referred to as microplastics. Even smaller plastics, measuring at a single micrometer, may be referred to as nano plastics. Such tiny plastic particles are believed to exist everywhere, from aquatic ecosystems in arctic polar regions (See Bergmann M., et al., 2017, "High quantities of microplastic in Arctic deep-sea sediments from the HAUSGARTEN observatory"), to bodies of land, in water (see Hu et al., 2018, "Microplastics in small waterbodies and tadpoles from Yangtze River Delta, China"), on beaches and coral reef systems (see Ding et al., 2019, "Microplastics in the coral reef systems from Xisha Islands of South China Sea,"), and even in marine animals (see Zhao et al., 2018, "Field-based evidence for microplastic in marine aggregates and mussels: implications for trophic transfer") and in humans (see Mastad, 2022, "Microplastic studies in humans: evidence from feces and effects in blood"). For example, Environmental International published research in April 2021, entitled "Placing nanoplastics in the context of global plastic pollution," noting accumulation of microplastic litter and microplastic fragments, discussing the burden of nanoplastics in various ecosystems. Conventional systems look to sediment to detect existence of microplastic particles (see Trinh et al., 2021, "A separation of plastic debris in Saigon river sediment using a microplastic sediment separator.")

### The State of Microplastics in 2023

An estimated greater than (>) 407 million tons of plastic is produced annually worldwide (Geyer et al. 2017), a number that is exponentially increasing (Jambeck et al. 2015), and an estimated 4.8-12.7 million metric tons of plastic enters the ocean every year (Jambeck et al. 2015). Plastics are found in virtually all aquatic environments sampled to date, including gyres (Goldstein et al. 2012), coastal waters (Gilfillan et al. 2009), shorelines (Browne et al. 2011), the deep sea (Van Cauwenberghe et al. 2013), rivers (Lechner et al. 2014), and the Great Lakes (Eriksen et al. 2013). Over 90% of plastic particles in the ocean are microplastics (Goldstein et al. 2013), generally defined as debris smaller than 5 mm in its longest dimension (Arthur 2009). There is a standing stock of 171 trillion pieces of microplastics floating in the ocean (Eriksen et al. 2023). The abundance of marine microplastics is also thought to be increasing, as Goldstein et al. (2012) found a two order of magnitude increase in abundance from 1972-1987 to 1999-2010 in the Northeast Pacific Subtropical Gyre, and Brandon et al. (2019a) found an exponential increase of microplastics in marine sediment from the end of WWII to 2010.

Although early research focused on microplastics in the environment, microplastics have now been found in tap or bottled water (Cox et al. 2019), sea salt (Karami et al. 2017), beer and soda (Shruti et al. 2020), fish (Atamanalp et al. 2021), tea (Hernandez et al. 2019), fruits and vegetables (Conti et al. 2020), and many other vectors for human consumption. More worryingly, microplastics have been found in human lungs (Jenner et al. 2022), blood (Leslie et al. 2022), stool samples (Schwabl et al. 2019), placenta (Ragusa et al. 2021), and breast milk (Ragusa et al. 2022), showing that we are exposed to microplastics even at our earliest stages of development. Although the effects of microplastics on human health is an area of active research, there are many documented health effects from the chemicals that are associated with plastics (UNEP 2021).

Even with all the current public attention being paid to microplastics, there are still many unanswered scientific questions and unsampled or undersampled regions around the globe, mainly due to the current inefficiency and difficulty in conventional techniques of sampling for microplastics.

### Current limiting factors in microplastic research

One limiting factor in microplastics research is that current methods used to collect and analyze microplastics are extremely tedious and time-consuming. Most often, conventionally, microplastics are collected via bongo or manta nets, water samples using Niskin bottles (an improvement on the Nansen bottle) or other water samplers, ship flow-through systems, or water pumps. Plastics from both net and water samples are often manually processed via density separation or filtration. The collected samples are then analyzed spectrally, via, e.g., fourier transform infrared (FTIR) spectroscopy, near infrared (NIR) spectroscopy, Raman spectroscopy, or fluorescence microscopy. All these methods require a large amount of time for both sample collection and analysis. There are currently no sensors that can give real time nor even near realtime concentrations of microplastics in a water sample. As all current methods require large amounts of labor hours and training, and as a result, many parts of the environment remain vastly understudied.

Another limiting factor is the cost of current used methods. Since these conventional methods are often designed to give highly specific readings of the polymer structure of each individual piece of microplastic, one at a time, the conventional methods are often overpriced and over-specified for the use case of detecting the presence/absence of plastics or overall concentration of plastics in a water sample.

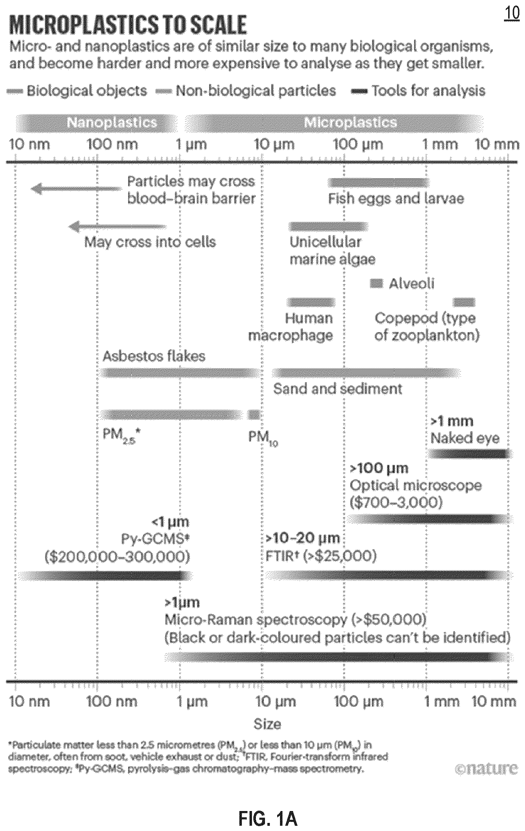

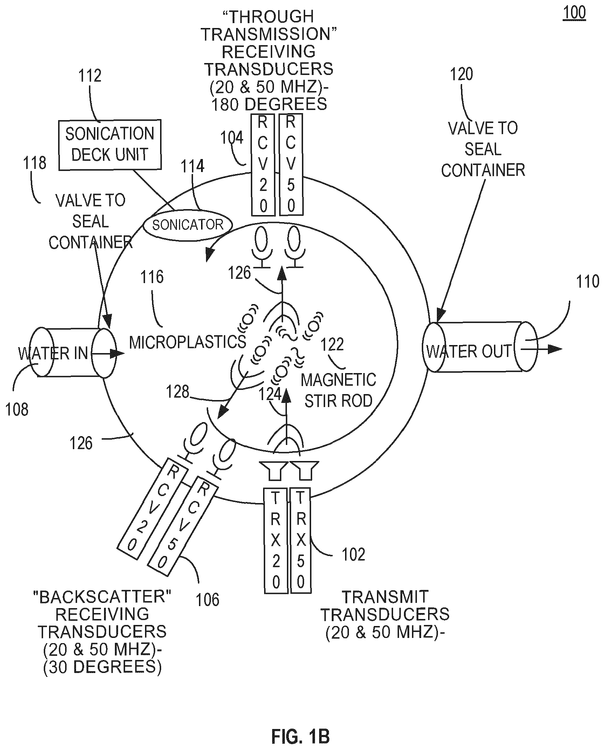

Microplastics increase in difficulty to detect as they get smaller in size, and different methods have varying limitations, such as, *e.g*., Raman spectroscopy, not being able to detect dark-colored particles (FIG. 1, from Lim 2021), etc. The different sizes of microplastics vary in ecological and human health importance, with the smallest microplastics being the most important to detect when concerned with human exposure, such as in drinking water (see FIG. 1 from Nature magazine).

### Unmet Needs

Example target customers include those who currently sample water, or who have the potential to be sampling water, for microplastics in, e.g., but not limited to, the academic, government, nonprofit, and water treatment spaces. Applicants have recognized, from customer discovery interviews and Applicant J. Brandon's personal experience, that many of the target groups want to sample, but are not sampling for, microplastics, or are not sampling frequently, due to various shortcomings of conventional sampling methods, which are exceedingly slow and tedious. Most often, microplastics are collected via bongo or manta nets, water samples using Niskin bottles, ship flow-through systems, or water pumps. Plastics from both net and water samples are conventionally manually processed via density separation or filtration. Samples are then analyzed spectrally, often particle by particle, via fourier transform infrared (FTIR) spectroscopy, near infrared (NIR) spectroscopy, Raman spectroscopy, or fluorescence microscopy. These methods require a large amount of time, on the order of hours to days per sample, for both sample collection and analysis, as well as significant training. Because of the complexity of these tests, samples often have to be sent out to labs for testing, which increases the time and cost to get results, making conventional solutions highly cost ineffective.

There are currently no sensors that can give real time concentrations of microplastics in a water sample.

Some conventional electromechanical sensing technologies for testing soil samples exist including membrane interface probes, which can capture data, to be later analyzed, such as those disclosed in US Patent 5,639,956, and US Patent 7,221,171, and the like, to perform chemical and other testing of soil subsurface, based terrestrial sampling measuring for given chemicals over a membrane. However, even such technologies fail to address the noted shortcomings of conventional testing solutions.

Conventional methods thus fail to provide for effective ways to detect existence of microplastics in substantially liquid samples. What is needed is an improved sampling technology, for achieving timely detection of existence of particular types of particles in aqueous and other primarily liquid samples, that overcomes shortcomings of conventional solutions.

Improved methods of ascertaining levels of plastic particles in largely liquid samples are needed that overcome the shortcomings of conventional solutions.

### Summary of the Invention

### Overview

According to one example embodiment, Applicant sets forth various example embodiments of a detection subsystem, which may include an ultrasound-based sensor configured to detect and characterize microplastics in a sample of fluid such as, e.g., but not limited to, water, according to an example embodiment. An example embodiment may include, e.g., but not limited to a sensing apparatus as available from Applied Ocean Sciences LLC (AOS), of 5242 Port Royal Road #1032 Springfield VA 22151 USA. AOS has confidentially demonstrated that it is possible to map scattered and transmitted ultrasonic signals in a sample of water to the concentration of microplastic particles in the sample, as well as distinguish between microplastics and organic matter, as set forth in various example embodiments disclosed herein, according to one example embodiment. AOS, according to one example embodiment, has designed an example commercial sensor that can be used by, e.g., but not limited to, scientists, drinking water providers, water treatment and monitoring facilities, conservation organizations, and human health professionals, etc., according to an example embodiment.

Various conventional electromechanical sensing technologies exist for detecting particles, however, none of those conventional solutions provide for detection of materials in a substantially liquid such as, e.g., but not limited to, water sample, according to an example embodiment.

### Intellectual Merit

Using ultrasound to detect particles suspended in a fluid is not new. For example, an engineer on the Applicant AOS team has various patents creating ultrasound-based sensing methods for bubbles in water, defects in fuel, and contaminants in blood, see US Patent Nos. , the contents of all of which are incorporated herein by reference in their entireties. One novel and nonobvious aspect of various example embodiments of the claimed invention is using ultrasound to detect plastic particles in a fluid, according to an example embodiment. Conventional ultrasonic particle detectors use reflection and scattering to detect things like bubbles in a liquid sample, based on different material bulk modulus of what the detector detects as compared to water. Unfortunately, this process does not work for plastics as the bulk modulus of many of polymer-based materials is like that of water. According to one example embodiment, Applicants present a novel detection method that takes advantage of multiple physical processes simultaneously to detect plastics in an example substantially liquid sample, according to one example embodiment. According to an example embodiment, embodiments of the present invention can measure spectral (e.g., but not limited to, frequency dependent, etc.) differences in ultrasound based on example characteristics including, e.g., but not limited to, backscatter, reflection, through transmission, attenuation, and/or resonance, etc., according to one example embodiment. When ultrasound travels through an illustrative example sample of water, according to one example embodiment, the ultrasound wave may, e.g., but not limited to, reflect and scatter off some particles, excite vibrations (resonance) in some particles, absorb energy at certain frequencies, and not at other frequencies, and may impact the bulk attenuation of the fluid by altering the viscous effects responsible for attenuation at these frequencies, according to one example embodiment. According to one example embodiment, by placing multiple example ultrasonic receivers in the example ensonification chamber, according to one example embodiment, an example system can capture all these exemplary effects, and may map the received signals, according to one example embodiment, *empirically,* to the concentration and composition of microplastics in the sample, according to one example embodiment.

### Example Broader Impacts of Various Example Embodiments

Human health implications of microplastic contamination in drinking water are alarming and a sensor, according to one example embodiment, that can detect the presence of microplastics in a water sample quickly and cheaply has the potential to further the national science foundation (NSF) broader impact goal of "advancing] the health and welfare of the American public," according to one example embodiment. In addition, social justice implications of this technology, according to one example embodiment, cannot be exaggerated. Everyone deserves clean drinking water, and various example embodiments of Applicants' claimed inventions provide various advantages over any conventionally available solution. The example technology, according to example embodiments, developed and set forth herein, as part of the SBIR to which this relates, is the most promising technology that shall provide a sufficiently affordable solution to communities everywhere, enabling monitoring of drinking water for plastic pollution, using technology according to various example embodiments. Prior to working on the various example embodiments of the disclosed plastics sensor, Applicant AOS's primary business has been in the defense and intelligence space, where Applicant has been aware of numerous applications for the principal technologies developed under this program, including detecting contamination in water or fuel supplies, and detecting buried explosive devices, and has applied that knowledge to develop various example embodiments set forth herein. During a phase of the Applicant AOS's research project, it has had two professors on the team as consultants and have used beta test sensors at two universities and multiple industrial facilities, in addition to prior testing already completed, to further define and improve on various other embodiments, according to example embodiments. The additional testing performed using the example partners, enhanced partnerships between academia and industry in the United States, according to example embodiments.

Applicant AOS intends to continue to grow a sustainable business building (in the U.S.) and selling sensors based on this technology, in alignment with the NSF goal of "increasing the economic competitiveness of the United States."

### Example Technology Overview According to Various Example Embodiments:

Applicant has created an example sensing device that can detect small particles of a substance suspended in a matrix using ultrasound sensor(s), according to some example embodiments.

Applicant has demonstrated that an example embodiment of the device can detect microplastic particles, and biological material, but the sensing device can also be used to detect other material such as suspended sediment, according to some example embodiments.

Applicant has demonstrated that the sensing device can be used to detect particles in a fluid such as, e.g., but not limited to, water, but the sensing device can also be used in other matrixes (or matrices) such as sediment or other complex structures, according to some example embodiments.

Example embodiments of the method may include, e.g., but not limited to, at least one ultrasonic transducer (which itself may include a transmitter, or a receiver, or a transmitter and a receiver), but can also include, in other example embodiments, multiple transducers, according to some example embodiments. Applicants have demonstrated example sensing devices systems including up to four example ultrasonic transducers, according to some example embodiments.

According to an example embodiment, ultrasonic energy may be transmitted into the sample containing the particles, according to one embodiment, and the ultrasonic energy may also be recorded in at least one location, according to an example embodiment.

The way that the ultrasound signal changes as the ultrasound signal travels in the sample may be empirically mapped to, e.g., but not limited to, the concentration and/or composition (including, e.g., but not limited to, size, shape, material properties, etc.), etc., of the particles in the matrix, according to an example embodiment.

Applicants, according to one example embodiment, have demonstrated an example system which may use an example receiving transducer, which may be mounted at an example acute angle to the example transmitting transducer, which may detect backscattered and other ultrasonic energy, according to an example embodiment.

Applicants have demonstrated an example system, according to an example embodiment, which may make use of an example receiving transducer, which may be mounted on the opposite side of the example transmitting transducer, according to an example embodiment, which may detect the transmission of ultrasonic energy through the sample, according to an example embodiment.

Any number of example transducers can be mounted in any combination of locations, according to various example embodiments of the claimed invention.

### Various example novel features of Example Embodiments

An example method is novel in that Ultrasound may be used to detect microplastic particles, according to an example embodiment.

The example method is novel in that the method, according to an example embodiment, can detect not only the number of particles (e.g., concentration or count) but also the composition including, e.g., but not limited to, size, shape, and material properties, according to an example embodiment.

The example method is novel in that it can be used, according to certain example embodiments, to detect particles in real-time whereas conventional systems require time-intensive analysis.

The example method is novel in that it can use empirically measured observations of the example ultrasonic signal in a sample, rather than an analytical or numerically derived relationship, in order to detect particles, according to an example embodiment. According to an example embodiment, a data-driven approach can be used, according to an example embodiment.

The example method can make use of example ultrasonic processing including, e.g., but not limited to, scattering, attenuation, transmission loss, travel time, resonance, reflection, refraction, and/or other physics, according to an example embodiment, involved in the interaction of ultrasound with a matrix containing particles, according to an example embodiment. The example processing can include, e.g., but not limited to, the particles reflecting, scattering, absorbing, resonating, and/or impacting the bulk properties of the matrix such as, e.g., but not limited to, frictional coefficients or viscosity, such as, e.g., but not limited to, the effects of suspended particle hydrophobicity on fluid bulk viscosity, according to an example embodiment.

Various example embodiments are directed to a method, system or apparatus configured to characterize or detect small plastics particles or similarly small particles, suspended in a fluid or a solid matrix, using acoustic or ultrasonic energy/waves, according to an example embodiment. Embodiments of the claimed invention, according to an example embodiment, may include, but are not limited to, methods making use of one, or any combination of, e.g., but not limited to, ultrasonic resonant spectroscopy, attenuation tomography, scatterometry, and/or reflection-based imaging techniques, etc. The specific example embodiment of the method demonstrated by Applicants may include, or comprise, an `ensonification chamber' with multiple ultrasonic transducers mounted around the perimeter, and along movable mounts, from which a variety of acoustic waveforms, including, but not limited to, pulses, continuous wave tones, sweeps, chirps, and M-sequences, etc., according to an example embodiment, may be transmitted and received, according to an example embodiment. The energy that is received, according to an example embodiment, on each example transducer may be indicative of the amount of energy that is reflected, transmitted, scattered, absorbed, and resonated by the plastic particles suspended in the media, according to an example embodiment. This information, according to an example embodiment, may then be mapped to concentration and composition, including, e.g., but not limited to, size, shape, type, and weathering state, etc., of plastics suspended in the media, according to an example embodiment. According to an example embodiment, an example UltraPlasticsSensor device can include an inexpensive, near real-time bulk concentration measurement device to provide a detected bulk concentration for a given sample, according to one example embodiment.

Various example embodiments of apparatuses, systems, and methods are set forth including, e.g., but not limited to, An apparatus configured to detect small particles of a substance suspended in a matrix using ultrasound, may include, e.g., but not limited to: a container device configured to receive a sample in an inner volume, said container device comprising: at least one first ultrasonic transducer on mechanically coupled to or disposed on at least one first exterior portion of said container device and oriented toward said inner volume of said container device, said at least one said first ultrasonic transducer may include: at least one first ultrasonic transmitter; and at least one first ultrasonic receiver; optionally at least one first receiver amplifier coupled to said at least one first receiver; optionally at least one first transmitter amplifier coupled to said at least one at least one first transmitter amplifier; at least one of: at least one software defined radio (SDR), or at least one signal generator and at least one oscilloscope, wherein said at least one software defined radio (SDR) or said at least one signal generator and said at least one oscilloscope are electronically coupled to said at least one first ultrasonic transmitter and electronically coupled to said at least one first ultrasonic receiver amplifier; and at least one electronic controller coupled to said at least one software defined radio (SDR) or said at least one signal generator and said at least one oscilloscope, according to one example embodiment.

According to an example embodiment, the container device further may include: at least one second ultrasonic transducer on at least one second exterior portion of said container device, oriented toward said inner volume of said container device, according to one example embodiment.

According to an example embodiment, the at least one second ultrasonic transducer is oriented across from said at least one first ultrasonic transducer, according to one example embodiment.

According to an example embodiment, the at least one second ultrasonic transducer is oriented at an acute angle from a transmission direction of said at least one first ultrasonic transducer, according to one example embodiment.

According to an example embodiment, the container device may include at least one or more of:
wherein said at least one first exterior portion and said at least one second exterior portion comprise portions of a continuous outer surface of said container device at least partially surrounding said inner volume; wherein said at least one first exterior portion and said at least one second exterior portion comprise a plurality of portions of an outer surface of said container device; wherein said at least one first exterior portion and said at least one second exterior portion comprise a plurality of portions of at least one outer wall of said container device; wherein said at least one first exterior portion and said at least one second exterior portion comprise a plurality of portions of at least one cylindrical portion of said container device; or wherein said at least one first exterior portion and
said at least one second exterior portion comprise a plurality of portions of at least one polygonal portion of said container device; wherein said at least one first exterior portion and said at least one second exterior portion comprise a plurality of semicircular portions of at least one circular portion of said container device; or wherein said at least one first exterior portion and said at least one second exterior portion comprise a plurality of wall portions of said container device about an exterior of said inner volume of said container device, according to one example embodiment.

According to an example embodiment, the container device may include a shape comprising at least one or more of: at least one spherical container; at least one polygon shaped container; at least one cylindrical container; at least one circular cylindrical shaped container; at least one circular cross-section shaped container; or at least one polygon cross-section shaped container, according to one example embodiment.

According to an example embodiment, the container device comprises at least one or more of:
at least one inlet; at least one outlet; at least one cavity for receiving the sample; at least one internal volume for receiving the sample; or at least one vacuum seal, according to one example embodiment.

According to an example embodiment, the at least one or more of: wherein said at least one said second ultrasonic transducer is oriented in a direction opposite of said at least one first exterior portion; wherein said at least one said second ultrasonic transducer is oriented in a direction opposite of said at least one said first transducer; wherein said at least one said second ultrasonic transducer is oriented at an angle relative to a transmission direction of said at least one said first transducer; wherein said at least one said second ultrasonic transducer is oriented at an angle of about orthogonal relative to a transmission direction of said at least one said first transducer; wherein said at least one said second ultrasonic transducer is oriented at an angle of about 90 degrees (o) relative to a transmission direction of said at least one said first transducer; or wherein said at least one said second ultrasonic transducer is oriented at an angle of about 45 degrees (o) relative to a transmission direction of said at least one said first transducer, according to one example embodiment.

According to an example embodiment, the at least four ultrasonic transducers, according to one example embodiment.

According to an example embodiment, the device may further include at least one or more of: at least one oscilloscope coupled to said at least one first transducer; or at least one oscilloscope to detect and graph at least one signal from said at least one first transducer, according to one example embodiment.

According to an example embodiment, the device may further include at least one stir mechanism, according to one example embodiment.

According to an example embodiment, the device may further include wherein said at least one stir mechanism comprises at least one magnetic stir rod, according to one example embodiment.

According to an example embodiment, the device may further include, wherein said at least one stir mechanism is configured to at least one or more of: maintain particles in suspension in the sample; stir contents of the sample; enable stirring of the sample; prevent settling of particles from the sample; prevent sediment from forming from the sample; or prevent separation of composition of mixture of the sample, according to one example embodiment.

According to an example embodiment, the device may further include at least one sonication mechanism, according to one example embodiment.

According to an example embodiment, the device may further include wherein said at least one sonication mechanism comprises at least one or more of: at least one sonication deck unit; or at least one sonicator.

According to an example embodiment, the device may further include wherein said at least one sonication mechanism is configured to at least one or more of: break up at least one material in the sample; break up at least one biological material in the sample; create a standing wave; sonicate material in the sample; or break up material in the sample, according to one example embodiment.

According to an example embodiment, the device may further include, wherein the sample comprises at least one or more of: water; water matrix; groundwater; groundwater matrix; fluid; fluid matrix; matrix; soil; soil matrix; soil water matrix; ocean water; lake water; river water; freshwater; drinking water; or municipal water, according to one example embodiment.

According to an example embodiment, a method of detecting at least one small particle of a substance suspended in a matrix of a sample using ultrasound, may include: where the method may include (a) transmitting at least one ultrasound signal, by at least one ultrasonic transmitter of at least one ultrasonic transducer, (transmitter/receiver), but can involve multiple transducers. According to an example embodiment, a method may include a system of up to four, wherein said at least one ultrasonic signal comprises ultrasonic energy is being transmitted into the sample containing the small particles, and wherein the ultrasonic energy is recorded in at least one location; (b) receiving received ultrasonic energy, by at least one ultrasonic receiver of at least one ultrasonic transducer; (c) empirically mapping said received ultrasonic energy, comprising: empirically mapping comprising (i) mapping at least one concentration, and (ii) mapping at least one composition (size, shape, material properties) of the at least one small particles in the matrix, according to one example embodiment.

According to an example embodiment, the method where the mapping of said at least one composition may include at least one or more of: 1) mapping at least one size property of the at least one small particle in the matrix; 2) mapping at least one shape property of the at least one small particle in the matrix; or 3) mapping at least one material property of the at least one small particle in the matrix, according to one example embodiment.

According to an example embodiment, the method where the at least one ultrasonic transmitter comprises a transmission direction, and where said at least one ultrasonic receiver comprises a receiving transducer mounted at an acute angle to the transmission direction of the said at least one ultrasonic transmitter, and further may include: detecting backscattered and other ultrasonic energy, by said at least one ultrasonic receiver transducer, which may detect backscattered and other ultrasonic energy, according to one example embodiment.

According to an example embodiment, the method where the at least one ultrasonic transmitter may include a transmission direction, and wherein said at least one ultrasonic receiver is mounted across from for opposite to the transmission direction of said at least one ultrasonic transmitter, and further may include: a. detecting transmission of ultrasonic energy through the sample, by said at least one ultrasonic receiver, according to one example embodiment.

According to an example embodiment, the method where the receiving transducer may be mounted on the opposite side of the transmitting transducer, which may detect the transmission of ultrasonic energy through the sample.

According to an example embodiment, the method where the at least one ultrasonic transmitter comprises a transmission direction, and wherein a plurality of said at least one ultrasonic receivers are mounted at, at least one or more of: a plurality of positions, across from, opposite from, or at an angle to, said transmission direction of said at least one ultrasonic transmitter, and further may include: detecting transmission of ultrasonic energy through the sample, by said plurality of said at least one ultrasonic receivers.

According to an example embodiment, the method including any number of transducers can be mounted in any combination of locations.

According to an example embodiment, the method where the at least one small particle of the substance comprises at least one or more of: micron size particulates; 1 micron up to 1.2mm sized particulates; sub micron particles; nano particles; greater than 1 micron but smaller than 2mm sized; under 20 micron sized particles; polyvinylchloride (PVC); polystyrene; Polyethylene terephthalate or polyethylene terephthalate (PET or PETE); other plastics; a plastic; a perfluoroalkyl, or a polyfluoroalkyl (PFAS) particle; a fluoropolymer coating particle; a nonstick cookware substance particle; a polytetrafluoroethylene (PTFE or TEFLON^{®}-branded) particle; a firefighting foam PFAS particle; a perfluorooctanoic acid (PFOA) particle; a perfluorochemicals (PFCs) particle; a perfluorooctane sulfonic acid (PFOS) particle; a perfluorinated alkylated substance (PFAS) particle; a perfluorononanoic acid (PFNA) particle;a perfluorobutane sulfonate (PFBS) particle; a perfluorohexane sulfonate (PFHxS) particle; hexafluoropropylene oxide dimer acid (HFPO-DA or C3 Dimer Acid or GENX^{®}-branded) particle; a packaging foam particle; a food packaging PFAS particle; a household item PFAS particle; a stain-resistant PFAS particle; a nonstick cookware PFAS particle; an outdoor gear durable water repellent coating PFAS particle; a plastic particle; a microplastic particle; or a particle of plastic of size on the order of 5mm, or smaller, in at least one direction, according to one example embodiment.

According to an example embodiment, the method where the at least one small particle of the substance comprises at least one or more of: microbial contaminants; chemical contaminants; viruses; a biological substance; a disseminated intravascular coagulation (DIC); a sediment inorganic matter(SIM); a sediment pore water using solid-phase micro-extraction and gas chromatography/mass spectrometry in selected ion monitoring mode (SPME/GCMS-SIM); a _sediment organic matter (SOM); polycyclic aromatic hydrocarbon (PAH); one or more plankton; phytoplankton; one or more red tide; or microbes, according to one example embodiment.

According to an example embodiment, the method where the at least one SDR or said at least one signal generator is configured to: generate the at least one ultrasonic signal, according to one example embodiment.

According to an example embodiment, the apparatus may include where the at least one electronic controller comprises at least one or more of: a microcontroller; a personal computer; a system on a chip (SOC); an ARM processor; an electronic computer processor; a multi-core computer processor; or a portable tablet based computer device, according to one example embodiment.

According to an example embodiment, the apparatus may include where the least one sensor comprising at least one or more of: analog processing; digital processing; ultrasonic resonant spectroscopy; attenuation tomography; scatterometry; or reflection-based imaging, according to one example embodiment.

According to an example embodiment, the apparatus may include where the ultrasonic energy comprises at least one or more of: acoustic energy; a pulse; a continuous wave tone; a sweep; a chirp; or an M-sequence, according to one example embodiment.

According to an example embodiment, the apparatus may include where the at least one ultrasonic receiver may include at least one or more of: an array of receiver transducers; a plurality of receiver transducers; a circumferential array of receiver transducers about a periphery of a chamber; an array of super high frequency receiver transducers; a rail comprising a plurality of receiver transducers; a moveable rail comprising an array of receiver transducers; a plurality of super high frequency transducers movable in relation relative to proximity to the at least one ultrasonic transmitter; an aluminum (AL) rail; a rail above a chamber; or an array of any of low frequency, high frequency, or super high frequency receiver transducers comprising at least one or more of: a 20-30 MHz receiver transducer; a 30-40 MHz receiver transducer; a 40-60 MHz receiver transducer; or a 60-80 MHz receiver transducer, according to one example embodiment.

According to an example embodiment, the apparatus may further include where the at least one or more of: at least one first receiver amplifier electronically coupled to said at least one first ultrasonic receiver; at least one first transmitter amplifier electronically coupled to said at least one first ultrasonic transmitter; at least one analog to digital converter configured to at least one or more of: convert an analog signal to digital signal, capture or store digital data in at least one storage device; at least one fourier transform system performing processing in frequency domain across all frequencies; at least one filter configured to at least one or more of: filter an analog signal; tuning out noise; tuning out sediment; or tuning out biological material; a pre-amplifier configured to amplify an analog signal; a sonicator; an indicator of sound energy; an integration/summation of particulate levels; a sonication deck unit; at least one intake valve; at least one ingress valve; at least one egress valve; at least one exit valve; or at least one physical chamber for housing a given sample comprising at least one or more of: an ensonification chamber; a spherical chamber; a cylindrical chamber; a pipe; a polygonal cross section chamber; an enclosed chamber;
a circular cross section chamber; or a cylindrical circular chamber, according to one example embodiment.

According to an example embodiment, the apparatus may further include a time synchronization between transmitting from the at least one transmitter, and receiving from the at least one receiver, according to one example embodiment.

According to an example embodiment, the apparatus may further include a plurality of said at least one receiver at a range of angles to a transmission direction of said at least one ultrasonic transmitter, wherein each of said plurality of said at least one receivers is situated at, at least one or more of: at an acute angle from transmission direction; at 0 degrees from transmission direction; at 90 degrees from transmission direction; at 30 degrees from transmission direction; at 60 degrees from transmission direction; at 45 degrees from transmission direction; at 180 degrees from transmission direction; at 270 degrees from transmission direction; at 360 degrees from transmission direction; or at an angle of between 0 and 360 from transmission direction, according to one example embodiment.

According to an example embodiment, the method may further include where the at least one or more of: calibrating based a control sample; receiving an analog signal and converting it into a digital signal; detecting stuff(particulates) in a matrix; digitizing (A/D conversion); filtering; detecting particulate matter processing for travel; analyzing, by spectroscopy; resolving; focusing or forming a coherent beam of sound; acoustic processing; processing by ultrasonic resonant spectroscopy; processing by attenuation tomography; processing by scatterometry; processing by reflection-based imaging; processing ultrasonic frequencies; processing high ultrasonic frequencies; processing super high ultrasonic frequencies; processing high ultrasonic frequencies of 1-20 MHz frequencies; processing super high ultrasonic frequencies of >20MHz and <80MHz; processing by a regression; processing sample and calibrated controlled sample at all frequencies; processing for through transmittance; processing for back scattering; processing filtering for plastics; processing reflection; processing for density; processing for impedance; processing for at least one bulk modulus property - seismo-accoustics (material scientists); processing for at least one medical property; statistical or heuristics processing; processing using artificial intelligence or machine learning processing; processing by machine learning techniques; processing using neural networks; or processing using convolutional neural networks, according to one example embodiment.

According to an example embodiment, the apparatus may include where the detector is configured to detect at least one or more of: microplastic particles; biological material; or suspended sediment, according to one example embodiment.

According to an example embodiment, the apparatus may include where the detector is configured to detect at least one or more of: particles in a fluid; particles in water; particles in stormwater; particles in saltwater; particles in groundwater; particles in drinking water; particles in ocean water; particles in water to be desalinated; contaminants in water; contaminants in wastewater; contaminants in drinking water; suspended sediment in drinking water; salt in drinking water; plankton in drinking water; contaminants in fuel; buried objects; improvised explosive devices in a surf zone; particles in a matrix; particles in sediment; or particles in a complex structure, according to one example embodiment.

### BRIEF DESCRIPTION OF DRAWING FIGURES DEPICTING SEVERAL ILLUSTRATIVE VIEWS OF EXAMPLE EMBODIMENTS

The foregoing and other features and advantages of the invention will be apparent from the following, more particular description of exemplary embodiments of the invention, as illustrated in the accompanying drawings. In the drawings, like reference numbers generally indicate identical, functionally similar, and/or structurally similar elements. The drawing in which an element first appears is indicated by the leftmost digits in the corresponding reference number. A preferred example embodiment is discussed below in the detailed description of the following drawings:
FIG. 1A depicts an example chart illustrating various example particulate matter example sizes, shown by size from a *Nature* magazine illustration;
FIG. 1B depicts an example schematic of one example embodiment of the example illustrative system in which an example, but nonlimiting, illustrative two different frequencies of transducers are mounted at an example, but nonlimiting, three example different locations to receive example through-transmitted and back scattered ultrasonic energy, according to one example embodiment;

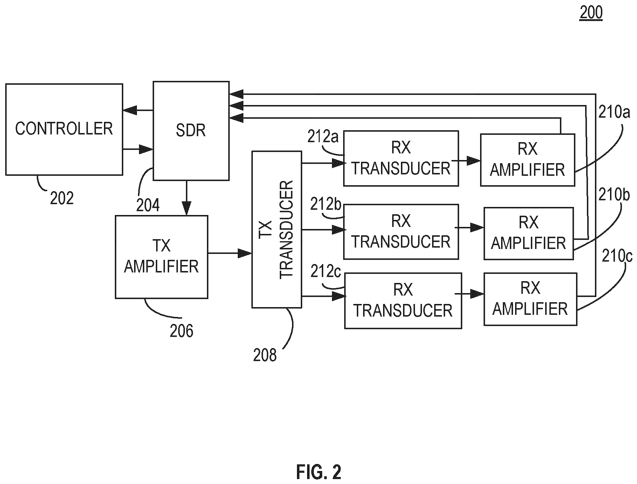

FIG. 2 depicts an example system diagram according to an example embodiment that includes, e.g., but not limited, a controller, a software defined radio, a transmitter amplifier and transducer, and a series of an example, but nonlimiting, three (3) receiving amplifiers and transducers, according to one example embodiment;

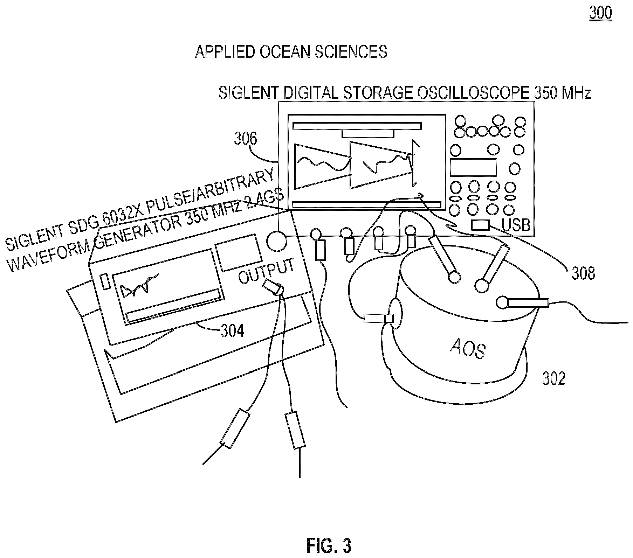

FIG. 3 depicts an example photo image of example embodiment of an illustrative system, according to one example embodiment;

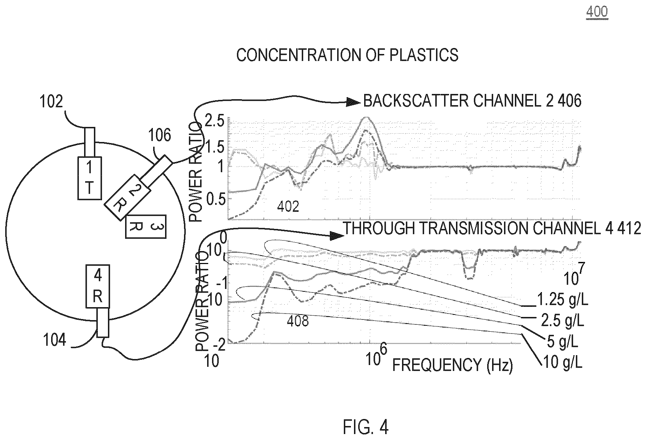

FIG. 4 depicts an example amplitude of backscattered and through-transmitted ultrasonic energy as a function of frequency (power spectra) of the example illustrative embodiment, showing a clear relationship to the concentration of microplastics in the example sample, according to one example embodiment, where an example of increasing concentration is demarcated by darker blue line color, according to one example embodiment;

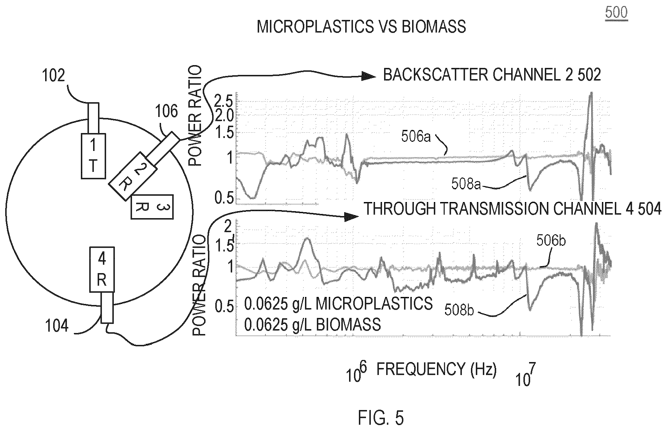

FIG. 5 depicts an example illustration of amplitude of backscattered and transmitted ultrasound as a function of frequency (power spectra) showing an example clear distinction between samples containing microplastics (shown in blue) and samples containing the same size distribution and mass of biological material (shown in purple), according to an example embodiment;

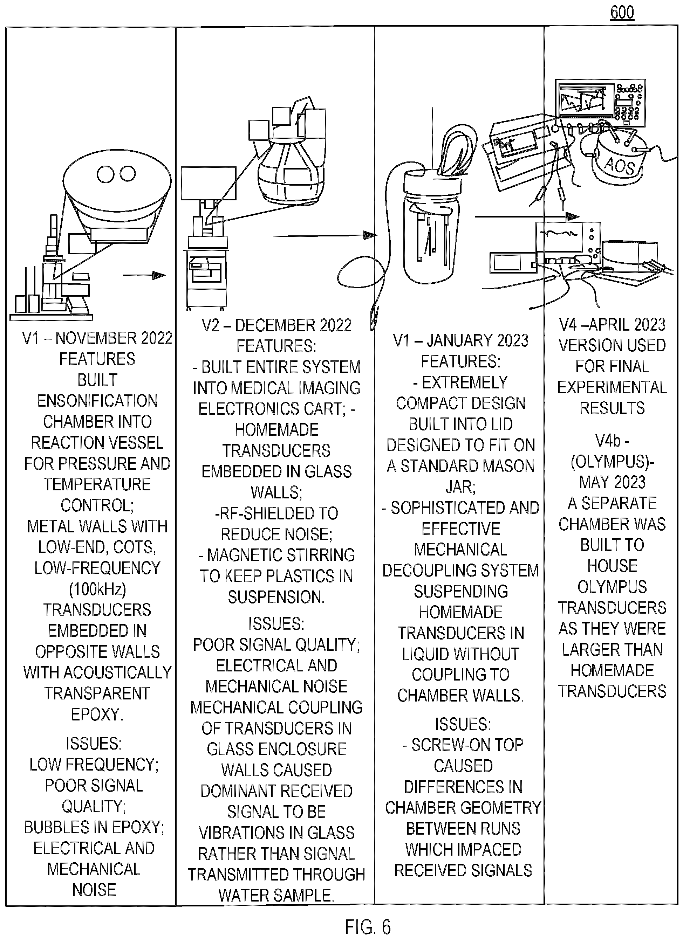

FIG. 6 depicting an example illustration of example evolution of example prototypes during Phase I, over various example stages of development, according to an example embodiment;

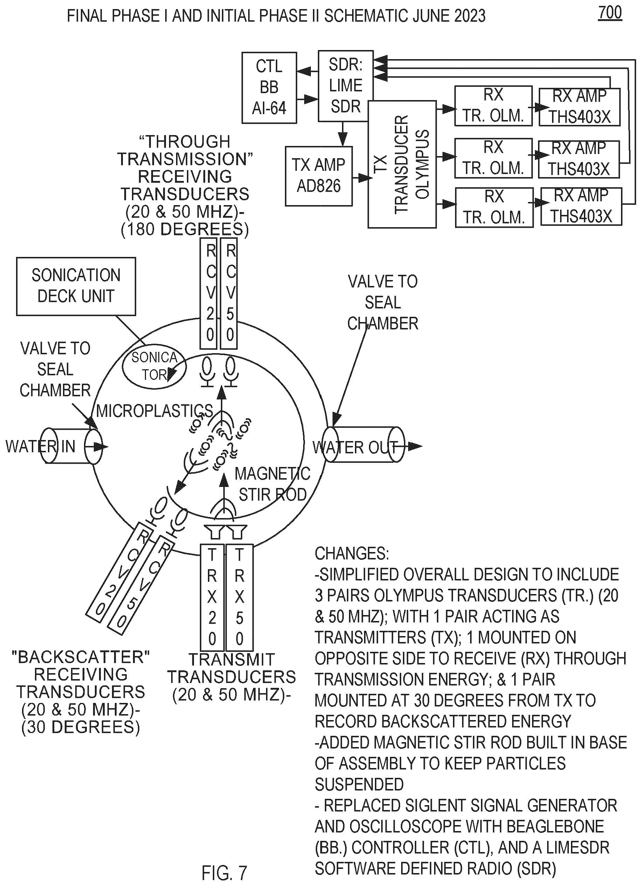

FIG. 7 depicts an example illustrative bird's eye view of an example schematic representation for an example sensor v5, the final Phase I design concept, including various example transducer sensors located at example angles of 30 and 180 degrees from the source transmitter transducer direction of transmission of example transmission of ultrasonic energy, rather than alternative examples of 45, 90, and 180 degrees as initially designed, according to one example embodiment;

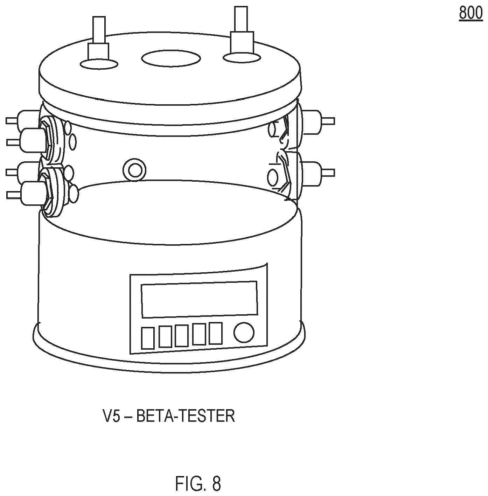

FIG. 8 depicts an example illustrative isometric three dimensional (3D) rendering of an example, but nonlimiting, example translucent, sensor v5, the Final Phase I design concept, along with an example top inlet and exit, and example transducers shown about an example circular cylindrical periphery, along with example integrated digital display illustrating an example graphical user interface depicting an example graphical chart of an example detected signal, according to one example embodiment;

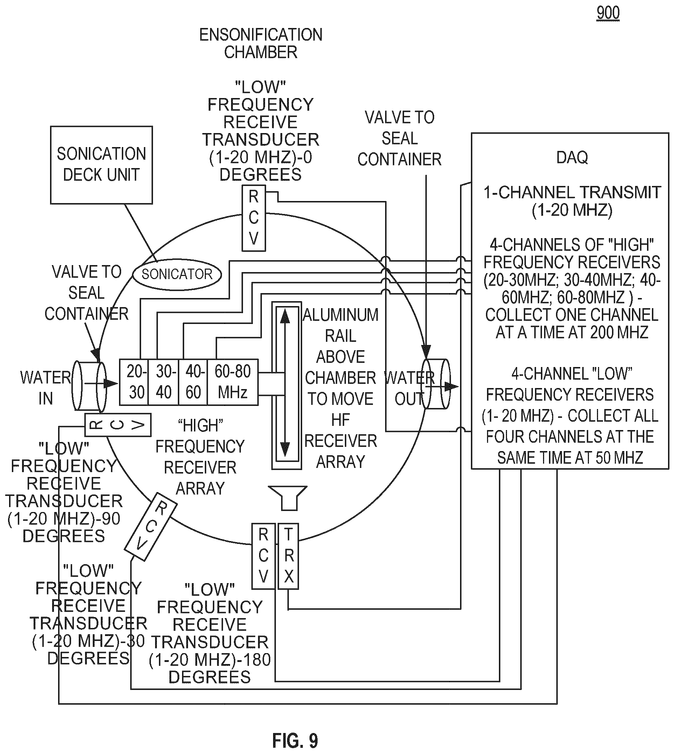

FIG. 9 depicts an example illustrative embodiment of an example ensonification chamber, according to one example embodiment; and

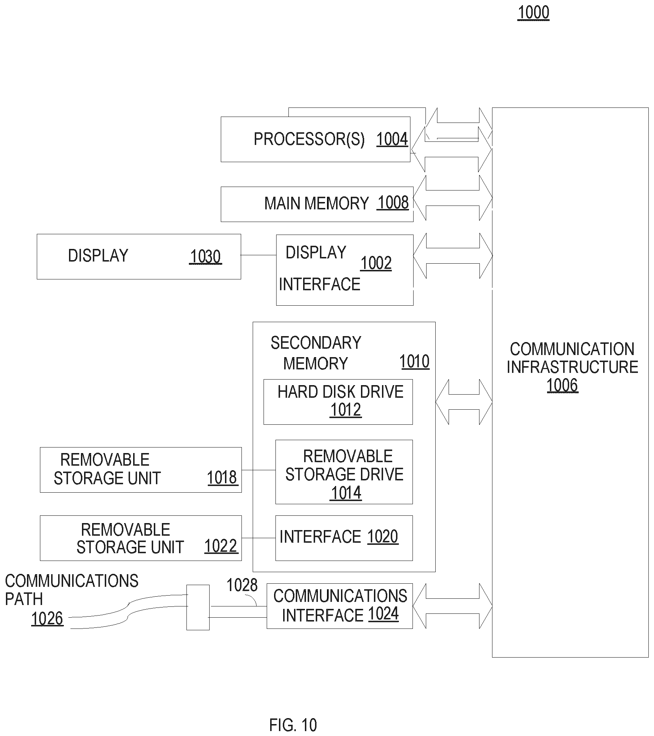

FIG. 10 depicts an example illustrative embodiment of an example block diagram of an electronic computer processor-based example controller as may be used in various example components, according to one example embodiment.

### DETAILED DESCRIPTION OF VARIOUS EXAMPLE EMBODIMENTS

FIG. 1A depicts an example chart 10 entitled "Microplastics to Scale," illustrating various example particulate matter example sizes, shown by size from a *Nature* magazine illustration.

FIG. 1B depicts an example schematic diagram 100 of one example embodiment of the example illustrative system in which, an example, but nonlimiting, illustrative two different frequencies (e.g., 20 and 50 MHz) of transducers 102, 104, 106, are mounted at an example, but nonlimiting, three example different locations (bottom transmitter transducers 102 in an upward illustrated transmission direction, transmitting ultrasonic energy 124, top receiver transducers 104 across from or opposite the bottom transmitters 102, across from and including example receiver transducers 104, described as an example 180 degrees position, and example backscatter receiver transducers 106, at an example 30 degrees from the transmission direction of the example transmitter transducers 102) example through-transmitted ultrasonic energy 126 and back scattered ultrasonic energy 128, according to one example embodiment.

FIG. 2 depicts an example system diagram 200 according to an example embodiment that includes, *e.g*., but not limited, an example controller 202, a software defined radio 204, a transmitter amplifier 206 and transmitter transducer 208, and a series of an example, but nonlimiting, three (3) receiving amplifiers 210 (210a, 210b, and 210c) and receiving transducers 212 (212a, 212b, 212c), according to one example embodiment.

FIG. 3 depicts an example photo image of example embodiment of an illustrative ultrasonic microplastics detector system, including an example sample testing chamber ultrasonic microplastics detector 302, coupled electronically to a software defined radio ultrasonic energy signal generator 304, and also coupled to an example digital oscilloscope 306 and digital storage device, including an illustrated example universal serial bus (USB)-coupled SDRAM or Flash memory storage device 308, according to one example embodiment.

FIG. 4 depicts an example illustration 400 graphing an example amplitude graph 402 of backscattered ultrasonic energy 404 of example channel 2 (406) illustrated, on an example logarithmic scale, and an example amplitude graph 408 of through-transmitted ultrasonic energy 410 for example channel 4 (412), both examples of sensed energy charted, as a function of frequency (power spectra) of the example illustrative embodiments, showing a clear relationship to the representative microplastic concentrations (see each example, solid line light gray 1.25 g/L, example light blue broken line 2.5 g/L, example solid light blue line 5g/L, and example broken dark blue 10g/L concentrations) of microplastics in the example sample, according to one example embodiment, where each incremental example of increasing concentration is demarcated by darker blue line color from light gray to dashed light blue, to solid blue, to dashed dark blue, according to one example embodiment.

FIG. 5 depicts an example illustration 500 of amplitude of backscattered 502 and transmitted 504 ultrasound as a function of frequency (power spectra) showing an example clear distinction between samples containing microplastics (shown in blue) 506 and samples containing the same size distribution and mass of biological material (shown in purple) 508, according to an example embodiment.

FIG. 6 depicting an example illustration 600 of example evolution of example prototypes during Phase I, over various example stages of development from V1, November 2022, to V2-December 2022, to V3 January 2023, to V4 April 2023, V4b May 2023, are illustrated reflecting graphical images of the example embodiments of each example prototype and illustrating the evolution of designs over time, and illustrating the early adoption and various alternative embodiments tested and evaluated for example effectiveness, according to an example embodiment.

FIG.7 depicts an example illustrative bird's eye view of an example schematic representation 700 for an example sensor v5, illustrating actual example commercial embodiments with commercial vendor devices illustrated including an example beaglebone AI-64 controller, LimeSDR software defined radio (SDR), example transmitter amplifier devices AD826, and Olympus Transmitter transducer, as well as Olympus receiver transducers and Receiver amplifiers THS403X example components, according to a June 2023 example embodiment, the final Phase I design concept, including example 20 MHz and 50 MHz transducers as illustrated, example magnetic stir rod, optional sonicator, example optional valves to seal entry and exit, example schematic of chamber, which is illustrated as an example circular cylinder, including various example transducer sensors located at example angles of 30 and 180 degrees from the source transmitter transducer direction of transmission of example transmission of ultrasonic energy, rather than alternative examples of 45, 90, and 180 degrees as initially designed, according to one example embodiment.

FIG. 8 depicts an example illustrative isometric three dimensional (3D) rendering 800 of an example, but nonlimiting, example translucent, sensor v5, the Final Phase I design concept, along with an example top inlet and exit for receiving an example sample, which may include optional valves and/or other example subsystems, not shown, including e.g., but not limited to, e.g., a sonicator, and/or a magnetic stir rod, etc., and example illustrated receiver transducers shown about an example circular cylindrical periphery, at example angles relative to an example transmitter transducer, along with example integrated digital display illustrating an example graphical user interface depicting an example graphical chart of an example detected signal, according to one example embodiment.

FIG. 9 depicts an example illustrative embodiment 900 of an example ensonification chamber, according to one example embodiment. FIG. 9 illustrates another example embodiment with example rail, which may be aluminum with one or an array of receivers, which may include high frequency (HF) receivers, which in an example embodiment may be deposed above or adjacent to the example ensonification chamber, and the example HF array may include one or more channels, or as shown in one example embodiment, various example channel data acquisition systems and/or processes, according to one example embodiment, as well as one or more example transmitter transducer(s), and one or more example low frequency receiver transducer(s), according to one example embodiment.

FIG. 10 depicts an example illustrative embodiment of an example block diagram 1000 of an example electronic computer processor-based example controller as may be used in various example components, according to one example embodiment.

### Results of the Phase I Project

Microplastics are near ubiquitous in public waterways and oceans, but their concentration, size, type, and spatial extent remain an intractable sensing problem. The program objective is to create an ultrasound-based sensor that can quickly and cheaply detect, quantify, and classify microplastics in a given sample of water. During Phase I we demonstrated that it is possible to map scattered and transmitted ultrasonic signals in a sample of water to the concentration of microplastic particles in the sample, as well as distinguish between microplastics and organic matter.

The primary obstacle to monitoring human exposure to and planning effective remediation strategies for global microplastics pollution is the laborious and costly nature of both collecting and processing water samples to determine concentrations of plastics. More data is needed on the concentrations and sources of microplastics throughout the environment, including point sources of pollution like wastewater effluent (Browne et al. 2011). The UltraPlasticsSensor device created and iterated upon during the Phase I program by the team at Applied Ocean Sciences LLC (AOS) has the potential to overcome these obstacles. Customer discovery interviews in Phase I helped us determine intended commercial applications to focus on. The original idea for the UltraPlasticsSensor device, according to an example embodiment, came from J. Brandon's experience as an academic researcher, and a need to sample oceanic water more quickly and efficiently. However, it quickly became apparent through interviews that there were much broader and pressing commercial applications outside of oceanography research in the water quality monitoring space, including drinking water, wastewater, stormwater, and desalination treatment plants. Once we learned of the new law that was passed by the California State Water Resources Control Board, making California the first place in the world to require monitoring of drinking water for microplastic contamination, starting in fall 2023, we then realized that drinking water was the most urgent market to tackle. Water quality monitoring will become the first commercial focus, with academic and nonprofit microplastic monitoring as a secondary market.

### Achievements

In Phase I, we developed the UltraPlasticsSensor and demonstrated that it is possible to discern the difference between clean water and water containing microplastic contamination using ultrasound transmitted from a single source received at two locations within a sample of water, one directly opposite the transmitter, one at a small angle to capture backscattered acoustic energy. The UltraPlasticsSensor consists of a cylindrical prism tank with four transducers. Given a source transducer, receiver transducers are mounted 45, 90, and 180 degrees away. The tank is filled with distilled water and rests on a magnetic stirrer. A representative mixture (microplastics or biomass) is weighed to achieve the desired plastic concentration given the volume of the tank, and then added. Given the variable density, shape, and hydrophobic nature of some of these particles, the magnetic stirrer is set to high, creating a small vortex and pulling plastic downward. Larger particles required manually stirring and/or scraping the side wall of the tank. When material appears to be well mixed in the fluid, the stirrer is set to low for trials to begin.

We collect data in the following benchtop sampling paradigm: A signal generator is connected to a source transducer along the vertical wall of a cylindrical prism tank. Receiver transducers are mounted at 45, 90, and 180 degrees from the source transducer, roughly backscatter, side scatter, and through transmission, respectively. The tank rests on a magnetic stirrer to prevent the particles from stratifying in the water column. Multiple sweeps are recorded on each receiver channel as well as from the signal generator at 250 Msps, from 100 kHz to 40 MHz, each sweep 0.1 seconds in length. A reference signal is recorded with clean water before adding any sample material. In post-processing, a peak finding method is used to detect sweeps automatically from the FFT of the time series. The power along each sweep and each channel is isolated and then averaged to form a broadband frequency response. The ratio of the power of a sweep with material to the power of the reference sweep provides a first order frequency dependent sensitivity to the effects of material type and concentration on that channel.

We were able to map the transmitted and backscattered energy across a range of frequencies to the concentration of microplastics contained in the sample (Figure 1). A frequency-dependent difference in power response is observed in backscatter and through transmission. While the backscatter does not respond linearly to concentration, the through transmission data shows clear signal separation where a higher concentration diminishes through transmission. There is potential to exploit the dip at 3 MHz to gauge concentration though that may also be a function of particle type, shape, or size. Achieving high quality backscatter data is still possible by increasing the beamwidth of the transducer and moving the backscatter channel closer to the source transducer. Trials with the Olympus transducers, which are even narrow beamwidth and have a cleaner power draw, show the ability to differentiate between 0 and 0.04 g/L of plastic concentration via through transmission, with linear signal separation at concentrations above 0.2 g/L.

See discussion of FIG. 4. FIG. 4 depicts an example graph 400 of amplitude of backscattered and through-transmitted ultrasonic energy as a function of frequency (power spectra) of the example illustrative embodiment, showing a clear relationship to the concentration of microplastics in the sample, according to one example embodiment, where it may be seen that an example increasing concentration is demarcated by darker blue line color, according to one example embodiment.

See discussion of FIG. 5. FIG. 5 depicts as noted above, graphs 500 charting example amplitudes of backscattered and transmitted ultrasound as a function of frequency (power spectra) showing a clear distinction between samples containing microplastics (blue) and samples containing the same size distribution and mass of biological material (purple), according to an example embodiment.

Additionally, we were able to demonstrate discernible differences between transmitted and backscattered ultrasonic energy in samples containing equal concentrations of approximately equal size distributions of representative biological material, designed to replicate plankton, algae and other suspended organic material in water, and samples containing the same concentrations of approximately equal size distributions of microplastics (Figure 2). For both backscatter and through transmission the power ratio from microplastics tends towards unity. The power ratio from biomass has distinguishable features. Nulls around 12, 24, and 28 MHz are consistent in both backscatter and through transmission. Both signals display peaks around 28 MHz. The last null and its associated peak may be a strong resonance and correlate with particle shape, size, and density. In addition, on the backscattered signal, there is a consistent diminished intensity between 1.2 and 7 MHz compared to microplastics. A convolution between the backscatter and transmitted signal would effectively differentiate between a microplastic or biomass dominated water sample. Ample features exist to explore and design an empirical sensitivity to scattering from microplastics or biomass in water.

The collective results conclusively demonstrate the validity of the basic scientific principles on which our microplastic sensor is based and validates the potential of this underlying technology for detecting and characterizing microplastic contamination in water, to include differentiating plastic from other material present in the sample.

Differences in scattered and transmitted energy between biomass and microplastics are likely due to differences in material bulk modulus, reflectivity, and density of the relative particles. An additional explanation is a reduction in viscous volume attenuation in the fluid containing suspended particulate plastics. At frequencies greater than 100 kHz, the predominant source of attenuation are viscous and shear effects. Given the low-Reynolds number at the scale of the particle interaction with ultrasound, the viscous effects are likely reduced by the presence of small 'lubricating' particulates in the fluid matrix. This effect is well documented in the case of small hydrophobic particles such as the plastics used in this experiment (Xu et al. 2013) suspended in a liquid at low-Reynolds number scales. This is a potentially exciting development, as this viscosity-altering effect is highly dependent on the size and density of suspended particles, meaning this effect might provide a pathway for determining the size and type of material in the suspension. This will require further analysis.

During Phase I we successfully demonstrated the ability to differentiate the acoustic response of microplastics compared to biological materials. We experimented with different sizes, shapes, and types of microplastic particles, but have not yet definitively demonstrated the device's ability to characterize the microplastic composition of a sample by plastic type or shape. We believe we will achieve that early in Phase II.

### Evolution of design

In the 18 months of this Phase I effort, we produced and improved upon the benchtop prototype sensor device and mapped the ultrasonic fluid material impulse response to microplastic concentration, which was our main goal. We iterated through five prototype devices and conducted over 500 experimental trials.

During Phase I we refined the proposed design concept and iterated through 5 prototype sensor devices (FIG. 6) before arriving at our final Phase I design (Figures 7 and 8). This design will be the beta-testing prototype used during Phase II. Supply chain shortages limited availability electrical components such as microcontrollers and single board computers (SBCs). For highly specialized components, like the Olympus brand ultrasonic transducers we purchased through Evident Scientific, these shortages and delays were especially problematic. We overcame this by designing and fabricating our own low-cost transducers in house using piezoelectric ceramics from StemInc. It took three months to get the transducers to be performant enough for our purposes, but because of this development work, we were able to conduct multiple rounds of tests before the Olympus transducers arrived. The experience we gained designing our own transducers during Phase I, will lead to ways we can include low-cost transducers for future versions of our sensor. Depending on the frequency range, the Olympus transducers cost between $1,000-$7,000, while we are now able to fabricate our own for less than $20 in components.

In June 2023 we completed the design of V5 (see FIG. 8), which was used for beta-testing during Phase II (see FIGs. 6 and 7). All electronics are mounted in the base of the unit. The chamber is built from 3d-printed parts and the same 150 mm acrylic tube used in V4 (see FIG. 6). Transducers can be mounted using universal bulkhead penetrator hardware. A touch screen and small camera are mounted in the lid to record images. We removed the 90o transducers, and mounted three pairs of transducers (20 MHz, 50 MHz) with receivers located at 30o and 180o, relative to the transmitter. We replaced the signal generator and oscilloscope with a BeagleBone controller and LimeSDR software defined radio, using the same amplifiers used in V4. The MATLAB signal processing code from Phase I was ported to C and run locally on a Raspberry Pi and displayed on the touch screen during Phase II. The unit has built-in sonicators, magnetic stirring, air compressors and water pumps.

### Customer Discovery

During Phase I, we conducted approximately 30 formal customer discovery interviews as part of the I-Corps `Beat-the-Odds Boot Camp,' and held dozens of informal discussions with colleagues and industry partners. During this process we discovered a beachhead market for our technology far more promising than our originally intended target. We initially intended to first market our technology to oceanographic researchers interested in studying microplastic pollution in the ocean. This market is probably limited to a few hundred organizations. While this remains an eventual target for us, we discovered that drinking water and wastewater treatment communities have a more pressing need for this technology and represent a much larger market. The state of California, for example, recently passed legislation that mandates that all drinking water be tested for microplastic contamination and experts agree other states will soon follow. It is also anticipated that desalinated water and wastewater will eventually have similar requirements. There are over 900 wastewater treatment plants, 12 desalination plants, over 400 major drinking water treatment systems, and well over 1,000 smaller systems in the state of California alone (California State Water Resources Control Board). The current technology to test this water is prohibitively expensive ($1000-$3000 per water sample) and requires multiple days to analyze a single sample of drinking water, making the prospect of sampling all drinking water in California not technically feasible. There is a need for a flow-through, near real-time system that can give a bulk abundance measurement of water samples before additional, time- and labor-intensive tests are conducted.

Fortunately, the requirements for a sensor that will meet the needs of the water treatment industry are far less technologically challenging than a sensor that will meet the needs of the scientific community. The scientific community requires a sensor that is robust, field deployable, small, and low-powered enough to be deployed aboard an autonomous vehicle at sea, as well as completely autonomous, capable of both measuring the precise concentration of microplastics in a water sample and characterizing the exact composition with regards to size, shape, and type of the plastics. Researchers operate on a tight budget so the cost of the sensor would need to be affordable (less than $30,000 according to our customer discovery interviews). While we believe we can meet this rigorous standard eventually, it is technologically very challenging and will likely take us two years to get there. The water treatment community, by contrast, requires only a bulk abundance measurement of microplastics that can be used to detect when plastics may be present in a water sample to prompt further precision testing. Drinking water also contains fewer interferers such as plankton, suspended sediment, or salt, which complicate measurements. Furthermore, the water treatment community does not require the sensor to be small, portable, or low-powered. A benchtop sensor would meet their needs fully.

In summary, through our customer discovery process, we learned that there exists a beachhead market two to three orders of magnitude larger than our original target market, with a lower technological barrier to entry. As a result, we have modified our main objective to first focus on analyzing microplastics in drinking water and wastewater. Our customer discovery interviews made it clear that there was the greatest first need for a benchtop water quality sensor to help detect microplastics in our water supply.

The sensor has the potential for immediate, broad, and urgently necessary societal impact by making possible a real-time alert system for the presence of microplastics in the drinking water supply. Furthermore, AOS is still firmly committed to making a sensor that is affordable to all communities and meets the needs of a variety of groups, including researchers and conservation groups. It is promising that while we build towards this goal, innovate to reduce the cost, and improve the capabilities of our sensor there is a current market for the technology. This market will in turn help fund this continuing development effort. NSF support has been, and remains, critical for our ability to embark on this research and development effort.

### Phase II Technical Objectives, Approach and Work Plan

The goal of this program is to create an ultrasound-based sensor that can detect and characterize microplastics in a sample of fluid such as water, according to an example embodiment. During Phase I we demonstrated that it was possible to map scattered and transmitted ultrasonic signals in a sample of water to the concentration of microplastic particles in the sample, as well as distinguish between microplastics and organic matter. AOS proposes, according to an example embodiment, to introduce a commercial sensor that can be used by scientists, drinking water providers, water treatment and monitoring facilities, conservation organizations, and human health professionals. During Phase II we shall have, according to an example embodiment,: (1) Quantified and automated the empirical relationship between ultrasonic transmission and the concentration and composition of microplastic particles present in a water sample; (2) Explored limits of the sensing methodology with respect to the size and minimum concentration of particles that can be detected; (3) Further explored the impact of interfering particles such as biological material or suspended sediment, including if the sensor can be used to classify these interferers as a warning system, e.g., for harmful algal blooms; (4) Developed five beta-testing prototypes, installed them on-site at partners' facilities, and conducted at least six months of extensive beta testing; (5) Refined the product into a finished commercial product, including custom analog circuitry and printed circuit boards (PCBs); (6) Engaged with a contract manufacturer to begin producing the finished product at scale; and (7) will have sold initial devices, and established a product sales, marketing, and technical infrastructure to better support our customers.

### Research Objectives

Two primary research objectives for Phase II are 1) to assess the lowest size threshold of microplastics that we can measure with ultrasonic acoustics, according to an example embodiment, and 2) to assess the lowest concentrations of microplastics that can be detected, according to an example embodiment.

The low size threshold is essential to analyze because microplastics <20 µm are the most harmful for drinking water and human health concerns. The fundamental physics on which our sensor is based are novel in that we combine classical ultrasonic reflection-based imaging with scatterometry, resonant spectroscopy, and bulk fluid tomography and interferometry. This means that the limitations of the method have not yet been fully characterized. Classical ultrasonic imaging and scatterometry can be used for particles down to about ½ the wavelength of the ultrasonic waves being used by the detector. For 20 MHz ultrasonic signal transmitted through water with a speed of sound of approximately 1500 m/s, the sensor could easily detect particles down to about 37 µm, according to an example embodiment. A 50 MHz signal, according to an example embodiment, could be used to detect particles down to about 15 µm. Our goal is to detect plastic particles down to 20 µm in size, where they become hard to detect by other means and more harmful to human health. Our data from Phase I and well-established ultrasonic imaging techniques indicate we will easily be able to accomplish this goal. Furthermore, initial Phase I results suggest that the through-transmission ultrasonic power spectra might be sensitive to the presence of far smaller particles, potentially down to below 1 µm in size. These smaller particles impact the attenuation coefficient and material vibrational modulus of the fluid, along with the sound speed; and they resonate at frequencies that are detectable. Acoustic attenuation at the frequencies we are working in, according to an example embodiment, is largely a function of shear and viscous effects, and suspended particles impact volumetric viscosity of a fluid in clear and dramatic ways. We may need to fully explore these phenomena during Phase II to establish the true bottom limit to the sizes of plastics we can detect, according to an example embodiment. Detecting smaller particles, according to an example embodiment, may require more expensive transducers and analog to digital converters, according to an example embodiment,. This may motivate us to create a "base" model sensor, which is affordable and can detect particles over 15 µm in size, and a "higher end" model, which costs more and can detect smaller particles, according to an example embodiment. Transducers are available off the shelf, without customization, up to 225 MHz that can easily allow us to detect down to 3 µm particles, and with more specialized custom hardware we can break 1 µm, according to an example embodiment,.

Low concentrations are essential to analyze because real world drinking water and open ocean samples often have much lower concentrations than laboratory tests. In Phase I we tested concentrations ranging from 0.0625 g/L to 10 g/L with the lowest concentration estimated to be to between 20,000 and 400,000 particles, which corresponds to the high end of published real-world concentrations (Mason et al. 2018). If the lowest detectable concentration exceeds that of the desired test concentration, the sensor may need to be modified, according to an example embodiment, to include a concentrating system such as an ultrasonic standing wave which can allow clean water to flow through yet restricts the flow of particles in the stream. This impacts the suitability of the sensor for certain applications, such as testing centers where it may be impractical to send large samples.

An important benchmark during Phase II can include testing the UltraPlasticsSensor, according to an example embodiment, on real world water samples of unknown microplastics types and concentrations, according to an example embodiment. We can address this, according to an example embodiment, through our partnership with the Southern California Coastal Water Research Project (SCCWR). SCCWRP can send us real world water samples that they have split. They can test one half of the sample with their current methods, and we will test one half of the sample with our device. This can allow us to have a blind sample set that we can then compare against a known analysis method and known measured abundance, according to an example embodiment. We can also plan to test our machine against real world seawater, wastewater effluent, and riverine samples, to examine the effects of unknown biologics and particles on our ultrasonic profiles. In year two of Phase II, we can require more sophisticated testing facilities with installed sensors, and facilities capable of replicating at sea conditions with greater fidelity. For this we can contract with Applied Research Associates (ARA) and make use of the test tank at Ohmsett - the National Oil Spill Response Research and Renewable Energy Test Facility in Leonardo, New Jersey. The Ohmsett facility is the largest outdoor saltwater wave/tow tank facility in North America and is 203 m long x 20 m wide x 2 m deep. Ohmsett's facility contains chlorinated saltwater, so we can test the sensor's performance with still saltwater as well as in choppy water, various sea states, and various amounts of bubbles simulating wake. We can also be able to simulate varying concentrations of microplastics in the test tank.

During Phase II we can conduct extensive beta testing, according to an example embodiment. In the first eight months of the program, according to an example embodiment" we can design and build an example five (5) beta-tester prototypes to be deployed at partner sites across the country. AOS can gather critical user feedback. These devices will be a transition between benchtop prototype and commercial product. We will primarily use COTS electronics, and multi-purpose micro-controllers, single board computers (SBCs), data acquisition systems (DACs), and analog to digital converters (ADCs). These beta-tester units can be equipped with 20 MHz and 50 MHz transducers, according to an example embodiment, capable of measuring microplastic particles down to 15 µm in size. We can deploy beta-tester prototypes at five sites around the country, according to an example embodiment, including SCCWRP in Los Angeles, SIO in San Diego, Ohmsett in Leonardo, New Jersey, and a wastewater treatment facility either in San Diego or Groton, Connecticut. We can assist on site personnel setting up the prototype sensors, conduct on-site training, and work closely with them for six months of extensive unit testing designed to test the limits of the system and gather valuable user and customer feedback on the operations and limitations of the system.

Throughout Phase II, we can be working to improve the performance of the AOS-made transducers, according to an example embodiment. We can optimize the resin used for potting the epoxies, according to an example embodiment. Classically acoustically transparent transducer encapsulant, according to an example embodiment, does not perform well for ultrasound because it may attenuate energy at high frequencies. We can experiment with several possible encapsulants, according to an example embodiment, until we identify the best one. Second, we can optimize the design of the housing, according to an example embodiment. This is important for beamwidth, vibrational isolation, and signal repeatability. We can begin, according to an example embodiment, by building the transducers into a plastic hull-penetrating fitting but may shift to stainless steel cases if they prove to be more performant. Next, we will experiment with different piezoelectric ceramics. In Phase I we used StemInc 1 MHz and 5 MHz ceramic disks, which performed well. There are a variety of sizes and thicknesses of these materials which impact the performance, according to an example embodiment. Finally, we will experiment with construction methods, such as how leads are affixed to the ceramic materials, and the method, according to an example embodiment, by which the ceramic is suspended in the encapsulant; we can work to optimize these design features through extensive experimentation, according to an example embodiment. A final design, according to an example embodiment, using AOS-made transducers can reduce the price of our finished product.

To test the limitations of the sensor with respect to size, concentration, and classification of microplastic contamination we will need a more sophisticated testing device. During the first six months of the program, we will design and build one highly specialized and sophisticated laboratory benchtop test system, which will be situated in Maryland. This system will be used to test the limits of the method, establish empirical relationships between desired measurement parameters, and conduct tests that will be useful to inform the design of our final system. This instrument is equipped with 5 MHz, 10 MHz, 20 MHz, 50 MHz, and 100 MHz ultrasonic transducers; uses a high-end signal generator and logging oscilloscope to transmit and receives a variety of waveforms and utilize lab-view software to manage the entire system.

The final technical objective of Phase II is to bridge the gap between beta-testing prototype and finished product. We have designed a final product instrument, according to an example embodiment. We are working closely with ARA and Vercet, two vendors to use for the design of the testing apparatus and custom analog circuitry and PCBs to complete the designs of a finished sensor instrument, according to other example embodiments. We are building this finished system, according to an example embodiment, using breadboards and protoboards as stand-ins for custom PCBs and planned to spend a month testing the system to work out the bugs. Finally, we are contracting an industrial fabrication company (contract manufacturer), and work on-site with this company to build five units of the finished product, according to an example embodiment, at their facility. This ensures a complete transfer of knowledge between our personnel and theirs. We are building one display unit, according to an example embodiment, for conferences and trade shows, one unit to remain at the AOS Maryland facility for testing and customer interaction, one unit to remain at the contract manufacturer site for reference, and two units, according to an example embodiment, to sell to our first beta-customers. In the six months after our 24-month Phase II effort, we are limiting sales to two units so we can build the product infrastructure required to support our product and our customers. The water testing community is small, and word of mouth is an important marketing tool; it is imperative that we fully support and ensure the success of our first two customers. After this six-month trial period, we are ramping up production and beginning selling units at scale.

### Commercial Objectives

Our first niche market was aimed to address is the California drinking water market. In September 2022, the California State Water Resources Control Board became the first in the world to pass regulations requiring drinking water to be monitored for microplastic contamination. Although the regulations specifically state that microplastics must be extracted and analyzed via FTIR and Raman spectroscopy (California State Water Resources Control Board 2022, Section 4.3.1), those methods are prohibitively costly and time-consuming. The policy handbook acknowledges this, stating that "costs and analysis time for microplastics analysis using the standardized methodologies are higher than many unregulated and regulated contaminants" (California State Water Resources Control Board 2022, Section 4.3.2). CalMatters.org, in reporting on the new regulations, states that the average cost per water sample would be $1000-$3000 (CalMatters.org, 07 September 2022).

The policy handbook also acknowledges that the study of microplastics is a new and fast-evolving field and states that any new analysis method can be adopted, but "the method in question must be validated through an inter-laboratory comparison exercise" (California State Water Resources Control Board 2022, Section 4.3.1). The UltraPlasticsSensor, according to an example embodiment, does not compete with Raman spectroscopy and FTIR for the level of specificity of analysis that they can provide. Rather embodiments of the present invention seek to help streamline the process by being the first step in the analysis process, analyzing the bulk abundance of microplastics in a sample. If a sample has an ultrasonic acoustic reading from our UltraPlasticsSensor, according to an example embodiment, indicating that it has high amounts of microplastics, it can then be analyzed via FTIR or Raman spectrometer, according to an example embodiment.

The need for such a step is addressed in Section 4.3.2 of the new California regulations, where the regulation says that there is "potential for inexpensive, rapid surrogate monitoring methods to indicate the presence of microplastics, which may be utilized to determine if additional monitoring using Raman or infrared spectroscopy is appropriate." Based on our customer discovery conversations with both Dr. Scott Coffin at the California State Water Resources Control Board and Steve Weisberg and Leah Thornton Hampton at Southern California Coastal Water Research Project (SCCWRP), it is clear that a real time ultrasonic sensor would likely be welcomed as a rapid surrogate monitoring step in this drinking water monitoring process, in order to handle the large volume of water samples that have to be analyzed for microplastics. All samples can be sampled via the UltraPlasticsSensor, and only those samples shown to have large abundances of microplastics can be analyzed under the more precise, expensive, and time-consuming methods of FTIR or Raman spectroscopy, according to an example embodiment.

Our first commercial objective, after prototyping the UltraPlasticsSensor, according to an example embodiment, with five beta-testers, and testing the UltraPlasticsSensor, according to an example embodiment, on real world water samples from SCCWRP, as outlined above, and improving the technology based on those results, can be to apply to be considered by the California State Water Resources Control Board as an official rapid surrogate monitoring method for drinking water, according to an example embodiment.

### Technical Approach:

The above research and commercialization objectives can be accomplished through the completion of an example 17 individual Tasks, planned. We can first do a thorough literature and regulatory review to ensure that we are up to date on how the rapidly changing microplastics landscape might have changed between the conclusion of Phase I and the beginning of Phase II. We will update our Phase II plan, given any significant developments. We can begin developing software and firmware immediately to run on the device, as this can be done in parallel with hardware development. We can begin by designing and constructing five beta-testing prototypes with the current V5 model as a starting point. We plan to have this completed and tested within the first eight months of the program. We can install these devices at four partner locations, keeping the fifth at the AOS Maryland facility for support and testing purposes. After six months of beta testing, we can collect all customer feedback during the beta-testing phase and use it to iterate on the beta-testing model, and conduct one additional, intensive round of testing of this improved beta-model at the ARA operated Ohmsett test facility. All of the lessons learned from these multiple rounds of testing can be taken into account as we design a finished system. This system can first be breadboarded with our partner vendors at ARA and Vercet, and once the system works the way it is intended, Vercet can design and fabricate the custom PCBs, while we work with ARA to design the physical test units, housings, and mechanical pieces at a contract manufacturer. We can iterate with the contract manufacturer, until we are satisfied with the finished product, then pivot to sales, marketing, and product support. Throughout the development effort we can be working with a sophisticated test unit at the AOS Maryland facility to refine algorithms and probe the limits of the technology. Finally, we can spend the entire two-years of Phase II working to improve the AOS in-house transducers, so that we can use them in the final product instead of the Olympus models. This can reduce the price of the finished product from $36,000 to $21,000 (with a 40% gross margin) making it available to a vastly larger potential user base.

### Broader Impacts

The human health implications of microplastic contamination in drinking water are alarming and a sensor that can detect the presence of microplastics in a water sample quickly and cheaply has the potential to further the NSF broader impact goal of "advanc[ing] the health and welfare of the American public." In addition, the social justice implications of this technology cannot be exaggerated. Everyone deserves clean drinking water. The technology we are developing as part of this SBIR is the most promising technology that can be sufficiently affordable that communities everywhere can monitor their drinking water for plastic pollution. Prior to working on this plastics sensor, AOS's primary business has been in the defense and intelligence space, where we are aware of numerous applications for the principal technologies developed under this program, including detecting contamination in water or fuel supplies, and detecting buried explosive devices. During Phase II of this program AOS has two professors on the team as consultants and we are planning to beta test sensors at two universities and multiple industrial facilities. AOS intends to grow a sustainable business building (in the U.S.) and selling sensors based on this technology, in alignment with the NSF goal of "increasing the economic competitiveness of the United States."

### Advancing the health and welfare of the American public

The first markets the UltraPlasticsSensor, according to an example embodiment, are focused on are drinking water and wastewater, which have both a human health and regulatory focus. Following that, we aim, according to an example embodiment, to expand into environmental and academic research markets with an in-situ model that can be used to monitor microplastics in all kinds of aquatic environments, from riverine to deep sea systems.

As our first target market, according to an example embodiment, the discipline of human health has far more potential use cases for the UltraPlasticsSensor, according to an example embodiment, than solely sampling drinking water. Microplastics have been found not only in tap and bottled water (Cox et al. 2019), but also sea salt (Karami et al. 2017), beer and soda (Shruti et al. 2020), fish (Atamanalp et al. 2021), tea (Hernandez et al. 2019), fruits and vegetables (Conti et al. 2020), honey (Liebezeit and Liebezeit 2015), and many other vectors for human consumption. More worryingly, it has been found in human lungs (Jenner et al. 2022), blood (Leslie et al. 2022), stool samples (Schwabl et al. 2019), placenta (Ragusa et al. 2021), and breast milk (Ragusa et al. 2022), showing that we are exposed to microplastics even at our earliest stages of development. Although the effects of microplastics on human health is an area of emerging research, there are many documented health effects from the chemicals that are associated with plastics (UNEP 2021).

There is a human health and safety need to sample more than just drinking water for microplastic. There is a need, according to an example embodiment, to sample agricultural water, both inputs and runoff, as well as food products like coffee, beer, wine, and soda. There is also a need, according to another example embodiment, to actually test human samples, like urine or blood, for microplastics. Although some of these human samples have been tested with traditional methods, the number of samples studied to date is very small due to cost and time limitations.

Microplastics increase in difficulty to detect, according to an example embodiment, as they get smaller in size, and different methods have varying limitations, such as Raman spectroscopy not being able to detect dark-colored particles. The different sizes of microplastics have differences in ecological and human health importance, with the smallest microplastics being the most important to detect when concerned with human exposure. The field of acoustics is well-equipped to deal with this problem, as different frequencies of transducers can be fine-tuned to test for different size particles. The sensing methodology developed as part of this SBIR effort relies upon reflection, scattering, ultrasonic resonant spectroscopy, and bulk fluid impedance changes arising from the presence of suspended plastic particulates. Further testing, according to an example embodiment, can be done to fully expound upon the limits in particle size detection associated with resonance, and bulk impedance measurements, but initial data suggests that it is possible to detect the presence of plastic particles much smaller than the wavelength of the ultrasound waves generated by the detector using these methods. For more traditional scatterometry and reflection imaging the rule of thumb is that it is possible to detect particles on the order of half the wavelength of the acoustic waves being used by the detector, according to an example embodiment. In other words, according to an example embodiment, according to an example embodiment, in a sample of water with a sound speed of 1,500 m/s, a 20 MHz source signal can be used to detect particles 37.5 µm in diameter; a 50 MHz signal can detect particles 15 µm in diameter; and a 100 MHz signal can detect particles 7.5 µm in diameter. As previously discussed, these estimates are for scattering mechanisms only, and as a result should be considered conservative, as the bulk impedance impacts of suspended particles appear to be more sensitive to smaller particles at lower frequencies than expected. To monitor drinking water for the purposes of most human health applications, it may be necessary to be able to detect particles less than 20 µm, which is possible with existing ultrasonic transducer technology, according to an example embodiment.

By creating an UltraPlasticsSensor, according to an example embodiment, that can sample the smallest size range of microplastics (0-20 µm), we can make an impact on the disciplines of human health, food safety, and medicine, by greatly expanding the knowledge base of how widespread microplastic contamination truly is, in terms of vectors of human consumption.

This increased knowledge, according to an example embodiment, can have the secondary impacts of leading to both food safety and medicinal regulations concerning microplastics, similar to the California drinking water microplastics regulations that have recently been legislated. It can also hopefully lead to more active research about the human health effects of microplastics and their associated chemicals and additives. Once these effects are more firmly established, regulations and restrictions around microplastics can likely quickly follow. Thus, a better, quicker monitoring system like the UltraPlasticsSensor, according to an example embodiment, may have far-reaching impacts in the long term far beyond just improved observational research.

### Improving public scientific literacy and engagement with science and technology in the United States

The UltraPlasticsSensor, according to an example embodiment, has the ability to vastly increase the number of microplastics samples taken, not only in drinking water and wastewater but also in rivers, oceans, lakes, and streams. Many local environmental monitoring groups and nonprofits monitor the water quality of their local water bodies, but they often only have the budget for the simplest of tests. Based on our customer discovery interviews and personal experiences collaborating with environmental nonprofits, they almost never have the budgets or the lab space to conduct today's current high-precision microplastic monitoring tests. However, giving them the first flow-through real-time sensor for microplastics can allow a level of water quality monitoring, environmental education, community engagement, and public education about microplastic pollution that has never been unlocked before. The in situ version of the UltraPlasticsSensor, according to an example embodiment, by being light, portable, and easy to use, can allow community members to easily partake in monitoring their local environments. This can allow them to see, in real time, the quality of their water and hopefully become engaged with the issue of microplastic pollution. After people see the issue up close, they often become engaged enough to want to help mitigate the issue in their local community, pass regulations on local polluters, and limit plastic usage in their own life. By increasing community outreach and knowledge on microplastics, and making the problem accessible to the community, the UltraPlasticsSensor, according to an example embodiment, can in turn engage local community stakeholders in helping change behavior and mediate the problem.

One of the most important impacts of the UltraPlasticsSensor, according to an example embodiment, is that it can allow more water to be sampled quickly and easily. This can have an enormous impact on currently undersampled waters in remote and underfunded regions, like the polar and equatorial ocean (van Sebille et al. 2015), as well as American water bodies that are severely undersampled for microplastics, like the Tijuana River estuary in San Diego. Because current sampling methods for microplastics requires net sampling or filtering water samples, and then analyzing those samples back in the lab under microscope with trained technicians, and/or using time-intensive and extremely extensive techniques like FTIR and Raman spectroscopy and scanning electron microscopy, the water that has been most sampled and analyzed for microplastics is near wealthy institutions of higher education, or the areas of most academic interest - the subtropical gyres. Whole other regions of the ocean and many rivers have been sampled once or twice, some not at all, and few conclusions can be drawn about their microplastic abundances with that little data.

The UltraPlasticsSensor, according to an example embodiment, can allow scientists and/or community groups in these regions quick and easy access to determine microplastic abundances for these undersampled and underrepresented areas and can allow these regions to be part of the larger global microplastic pollution discussions. During phase II we have partnered with the Walter Munk Foundation for the Oceans to conduct education and outreach activities with local San Diego schools using our sensor technology.

### Expanding participation of underrepresented groups in STEM and Developing an American STEM workforce that is globally competitive

An intended impact of the UltraPlasticsSensor, according to an example embodiment, is to significantly broaden participation in science and engineering by making microplastic sampling cheaper, easier, and faster, thus allowing more stakeholders to participate. This can allow engagement with microplastics and acoustics by women and individuals from underrepresented groups in STEM, while also developing an American STEM workforce through improved undergraduate STEM education and instruction.

The UltraPlasticsSensor, according to an example embodiment, can broaden participation to an entire range of labs, classes, faculty, and students that want to incorporate microplastics in their research programs and teaching curricula but currently cannot due to financial or time constraints. Broadened participation can include undergraduates and those at research institutions that are underrepresented or underfunded in terms of typical laboratory and field science equipment. While these students and faculty can currently take visual samples, or use a dissecting microscope, they cannot sample the smallest microplastics due to equipment constraints - e.g., the prohibitive costs of acquiring an FTIR or Raman spectrometer.

Beyond the classroom and academic setting, the UltraPlasticsSensor, according to an example embodiment, can also expand participation in microplastic and water monitoring by nonprofits, conservationists, monitoring groups, and other community stakeholders. By being a near real-time sensor, these groups will be able to quantify the microplastic concentrations in their local waters at any given time, for the first time ever. With current analysis methods there is at least a week or more wait time between when a sample is taken and when the microplastic concentration in that sample is known. If microplastics concentrations can be known in near real time, point sources of pollution can be better isolated, the effects of weather, waves, and other environmental factors on microplastics concentrations can be better understood and more areas, especially areas of public interest and benefit, can be sampled.

By allowing water to be sampled quickly and easily, it can give local stakeholders more ownership of the sampling process, and thus lead to more ownership over the remediation and mitigation process. If local community members see with their own eyes how contaminated their local water bodies are in real time, using embodiments, according to an example embodiment, they can have more motivation to help pass legislation and regulatory policies to enact change to clean that water and stop microplastic pollution at the source. The ability to expand public sampling of microplastic should directly lead to an increase in public interest and public engagement in the problem of microplastic pollution. Most of these impacts are promised by use of embodiments of Applicant's claimed inventions, according to an example embodiment, However, our customer discovery interviews for Phase I and Phase II with community stakeholders, especially those that work in the nonprofit space, lead us to believe that a faster, cheaper microplastics sensor, according to an example embodiment, can have an incredible impact on expanding microplastics sampling in local waterways, expanding community participation, and expanding access and exposure to multiple community stakeholders to the issue of microplastic pollution.

### Supporting the national defense of the United States

Prior to this project, most members of the AOS team worked primarily in the defense and intelligence space. We are aware of a multitude of applications of the technology we are developing here in the defense community. The technology can be used to detect contaminants in drinking water or fuel which is critical to forward deployed military operations. The core technology, and exploitation of a combination of acoustic scattering, resonance, and attenuation is applicable to other frequencies of acoustic energy and can be used for detecting buried objects such as improvised explosive devices, especially in challenging environments such as the surf zone, according to other example embodiments. This is a problem of paramount importance to the Marine Corps currently. The imaging technique can be used for detecting faults, defects, and cracks in complex matrices such as the carbon composite materials used in military airframes, propellers, and submarine hulls, according to other example embodiments. The technology can be adapted for doing non-destructive testing on these materials, according to other example embodiments. Furthermore, the algorithms and signal processing methodology can be applied to other sensing modalities such as ultrawideband micro-pulsed radar, according to other example embodiments, which can be used to image through solid barriers, and a variety of optical imaging techniques. The method by which we are empirically mapping the channel impulse response of the sample fluid for detecting microplastics in this program, shows enormous potential for multimodal sensor fusion which is a holy grail for military remote sensing technologies, according to other example embodiments. We have already discussed, under nondisclosure agreement, the technology and approach developed during phase I with program managers at DARPA and the Office of Naval Research, and they have expressed interest in being kept up to date on our progress and have inquired about several of the defense related applications described in this section, according to an example embodiment.

### Enhancing partnerships between academia and industry in the United States

Our Phase II team involves members of industry and academia, according to an example embodiment. We intend to beta-test our prototypes at Scripps Institution of Oceanography (SIO), and potentially the United States Coast Guard Academy; as well as at least one wastewater treatment plant, and the industrial test facility, Ohmsett, operated by ARA. We have received letters of support from academics, government organizations, and members of industry. Dr. Dimitri Deheyn, as a consultant on this project, and a professor at SIO, has already worked with members of the water treatment industry to make arrangements for beta-testing our prototypes and bringing local high school students to the water treatment facilities to conduct education and outreach activities. This project has already had an impact on enhancing the relationship between academia and industry, and we are confident this impact will grow in Phase II.

### Increasing the economic competitiveness of the United States

Applied Ocean Sciences intends to create a business by building and selling sensors, according to an example embodiment, based on the technology developed here. The instruments can be manufactured in the United States, with the custom PCBs sourced in Texas, according to an example embodiment. All of our partners, vendors, consultants, and contractors are U.S. based. The technology we are developing is critically needed all over the world, with the rising awareness of microplastics as a global threat. As a U.S. company, AOS developing, patenting, producing, and selling this globally critical technology can increase the economic competitiveness of the United States in the global technology sector.

### Important References upon which example embodiments may build

Applicant Applied Ocean Sciences directs the reader for further information to the following references useful in further understanding example embodiments of the claimed invention, according to an example embodiment:
American Membrane Technology Association, 2018: Future of Desalination in the United States. https:/ /www.amtaorg.com/wp-content/uploads/11_Future_of_Desal.pdf
American Society of Civil Engineers: 2021 Report Card for America's Infrastructure - Drinking Water. https://infrastructurereportcard.org/wp-content/uploads/2017/01/Drinking-Water-2021.pdf
American Society of Civil Engineers: 2021 Report Card for America's Infrastructure - Stormwater. https://infrastructurereportcard,org/wp-content/uploads/2020/12/Stormwater-2021.pdf
American Society of Civil Engineers: 2021 Report Card for America's Infrastructure - Wastewater. https://infrastructurereportcard,org/wp-content/uploads/2020/12/Wastewater-2021.pdf
Arthur, C., Baker, J., and Bamford, H. (Eds.), 2009: Proceedings of the International Research Workshop on the Occurrence, Effects, and Fate of Microplastic Marine Debris, September 9-11, 2008, University of Washington Tacoma, Tacoma, WA, USA. U.S. Dept. of Commerce, National Oceanic and Atmospheric Administration, National Ocean Service, Office of Response & Restoration, Silver Spring, Md.
Atamanalp, Muhammed, and Coauthors, 2021: Microplastics in tissues (brain, gill, muscle and gastrointestinal) of Mullus barbatus and Alosa immaculata. Archives of Environmental Contamination and Toxicology, 81, 460-469.
Brandon, J. A., W. Jones, and M.D. Ohman, 2019a: Multidecadal increase in plastic particles in coastal ocean sediments. Science Advances, 5(9), eaax0587.
Browne, M. A., P. Crump, S. J. Niven, E. Teuten, A. Tonkin, T. Galloway, and R. Thompson, 2011: Accumulation of microplastic on shorelines worldwide: sources and sinks. Environmental Science & Technology, 45, 9175-9179.
California State Association of Counties, "Inflation Reduction Act Summary." Accessed 19 June 2023, https://www.counties.org/post/inflation-reduction-act-summary#:~:text=The%20legislation%20will%20provide%20%24550, construction%20o f%20eligible%20water%20projects.
California State Water Resources Control Board, 2022: Policy Handbook Establishing a Standard Method of Testing and Reporting of Microplastics in Drinking Water. 26 pp.
CalMatters.org. Becker, Rachel, 2022: California approves microplastics testing of drinking water sources. https://calmatters.org/environment/2022/09/california-microplastics-testing-drinking-water-sources/.
Conti, Gea Oliveri, and Coauthors, 2020: Micro-and nano-plastics in edible fruit and vegetables. The first diet risks assessment for the general population. Environmental Research, 187, 109677.
Cox, Kieran D, and Coauthors, 2019: Human consumption of microplastics. Environmental Science & Technology, 53(12), 7068-7074.
Edson, Ethan C., and Mark R. Patterson, 2015: "MantaRay: a novel autonomous sampling instrument for in situ measurements of environmental microplastic particle concentrations." OCEANS 2015-MTS/IEEE Washington, Washington, DC, USA, 1-6, doi: 10.23919/OCEANS.2015.7404541.
Eriksen, M., and Coauthors, 2013: Microplastic pollution in the surface waters of the Laurentian Great Lakes. Marine Pollution Bulletin, 77, 177-182.
Eriksen, M., and Coauthors, 2023: A growing plastic smog, now estimated to be over 170 trillion plastic particles afloat in the world's oceans-Urgent solutions required. PLOS One, 18, e0281596.
Geyer, R., J.R. Jambeck, and K. L. Law, 2017: Production, use, and fate of all plastics ever made. Science Advances, 3(7), e1700782.
Gilfillan, L. R., M. D. Ohman, M. J. Doyle, and W. Watson, 2009: Occurrence of plastic micro-debris in the southern California Current system. California Cooperative Oceanic Fisheries Investigations Reports, 50, 123-133.
Goldstein, M. C., M. Rosenberg, and L. Cheng, 2012: Increased oceanic microplastic debris enhances oviposition in an endemic pelagic insect. Biology Letters, 8, 817-820.
Goldstein, M. C., A. J. Titmus, and M. Ford, 2013: Scales of spatial heterogeneity of plastic marine debris in the northeast Pacific ocean. PLOS One, 8, e80020.
Hernandez, Laura M., and Coauthors, 2019: Plastic teabags release billions of microparticles and nanoparticles into tea. Environmental Science & Technology, 53(21), 12300-12310.
International Institute of Sustainable Development, 2023: Summary of the Second Meeting of the Intergovernmental Negotiating Committee to Develop an International Legally Binding Instrument on Plastic Pollution: 29 May - 2 June 2023. https://enb.iisd.org/sites/default/files/2023-06/enb3612e.pdf
Jambeck, J. R., and Coauthors, 2015. Plastic waste inputs from land into the ocean. Science, 347, 768-771.
Jenner, Lauren C., and Coauthors, 2022: Detection of microplastics in human lung tissue using µFTIR spectroscopy. Science of the Total Environment, 831, 154907.
Jones, Edward, and Coauthors, 2019: The state of desalination and brine production: A global outlook. Science of the Total Environment, 657, 1343-1356.
Karami, Ali, and Coauthors, 2017: The presence of microplastics in commercial salts from different countries. Scientific Reports, 7(1), 46173.
Lechner, A., and Coauthors, 2014. The Danube so colourful: A potpourri of plastic litter outnumbers fish larvae in Europe's second largest river. Environmental Pollution, 188, 177-181.
Liebezeit, G. and E. Liebezeit, 2015: Origin of synthetic particles in honeys. Polish Journal of Food and Nutrition Sciences, 65(2), https://doi.org/10.1515/pjfns-2015-0025.
Leslie, Heather A., and Coauthors, 2022: Discovery and quantification of plastic particle pollution in human blood. Environment International, 163: 107199.
Lim, X, 2021: Microplastics are everywhere-but are they harmful. Nature, 593, 22-25.
Mason, Sherri A., Victoria G. Welch, and Joseph Neratko, 2018: Synthetic polymer contamination in bottled water. Frontiers in Chemistry, 6, https://doi.org/10.3389/fchem.2018.00407.
Ragusa, Antonio, and Coauthors, 2021: Plasticenta: First evidence of microplastics in human placenta. Environment international, 146, 106274.
Ragusa, Antonio, and Coauthors, 2022: Raman microspectroscopy detection and characterisation of microplastics in human breastmilk. Polymers, 14(13), 2700.
Schwabl, Philipp, and Coauthors, 2019: Detection of various microplastics in human stool: a prospective case series. Annals of internal medicine, 171(7), 453-457.
Shruti, V. C., and Coauthors, 2020: First study of its kind on the microplastic contamination of soft drinks, cold tea and energy drinks-Future research and environmental considerations. Science of the Total Environment, 726, 138580.
United Nations Environment Programme, 2021: From Pollution to Solution: A global assessment of marine litter and plastic pollution. Nairobi.
United States Environmental Protection Agency, Office of Environmental Information, 2020: EPA Facility Registry Service (FRS): Wastewater Treatment Plants. Washington, DC, USA. Retrieved from https://edg.epa.gov/data/PUBLIC/OEI/OIC/FRS Waterwater.zip. Accessed through Resource Watch, June 2023: www.resourcewatch.org.
United States Environmental Protection Agency, 2023: Bipartisan Infrastructure Law: Water Infrastructure Investments. Accessed 03 May 2023. https://www.epa.gov/infrastructure/water-infrastructure-investments
United States Environmental Protection Agency, National Pollutant Discharge Elimination System (NPDES), 2023: Combined Sewer Overflow Frequent Questions. Last updated June 2023. https://www.epa.gov/npdes/combined-sewer-overflow-frequent-questions
United States Environmental Protection Agency, 2023: "National Summary of State Information." Accessed June 2023 through "2021 Report Card for America's Infrastructure - Stormwater".
van Cauwenberghe, L., A. Vanreusel, J. Mees, and C. R. Janssen, 2013: Microplastic pollution in deep-sea sediments. Environmental Pollution, 182, 495-499.
van Sebille, E. and Coauthors, 2015: A global inventory of small floating plastic debris. Environ. Res. Lett., 10, 124006.
White House, 2023: "Fact Sheet: Biden-Harris Administration Announces New Investments to Protect the Colorado River System." Accessed April 2023, https://www.whitehouse.gov/briefing-room/statements-releases/2023/04/06/fact-sheet-biden-harris-administration-announces-new-investments-to-protect-the-colorado-river-system/.
Xu, M. and Coauthors, 2013: How to decrease the viscosity of suspension with the second fluid and nanoparticles? Sci. Rep., 5(3), 3137, https://doi.org/ 10.1038/srep03137.

### Glossary of Relevant Technology

**Digital signal processing (DSP)** is the use of digital processing, such as by computers or more specialized digital signal processors, to perform a wide variety of signal processing operations. The digital signals processed in this manner are a sequence of numbers that represent samples of a continuous variable in a domain such as time, space, or frequency. In digital electronics, a digital signal is represented as a pulse train, which is typically generated by the switching of a transistor.

Digital signal processing and analog signal processing are subfields of signal processing. DSP applications include audio and speech processing, sonar, radar and other sensor array processing, spectral density estimation, statistical signal processing, digital image processing, data compression, video coding, audio coding, image compression, signal processing for telecommunications, control systems, biomedical engineering, and seismology, among others.

DSP can involve linear or nonlinear operations. Nonlinear signal processing is closely related to nonlinear system identification and can be implemented in the time, frequency, and spatio-temporal domains.

The application of digital computation to signal processing allows for many advantages over analog processing in many applications, such as error detection and correction in transmission as well as data compression. Digital signal processing is also fundamental to digital technology, such as digital telecommunication and wireless communications. DSP is applicable to both streaming data and static (stored) data.

**Sampling (signal processing)** may include digitally analyzing and manipulating an analog signal, where the signal may be digitized with an analog-to-digital converter (ADC). Sampling is usually carried out in two stages, discretization and quantization. Discretization means that the signal is divided into equal intervals of time, and each interval is represented by a single measurement of amplitude. Quantization means each amplitude measurement is approximated by a value from a finite set. Rounding real numbers to integers is an example.

The Nyquist-Shannon sampling theorem states that a signal can be exactly reconstructed from its samples if the sampling frequency is greater than twice the highest frequency component in the signal. In practice, the sampling frequency is often significantly higher than this. It is common to use an anti-aliasing filter to limit the signal bandwidth to comply with the sampling theorem, however careful selection of this filter is required because the reconstructed signal will be the filtered signal plus residual aliasing from imperfect stop band rejection instead of the original (unfiltered) signal.

Theoretical DSP analyses and derivations are typically performed on discrete-time signal models with no amplitude inaccuracies (quantization error), "created" by the abstract process of sampling. Numerical methods require a quantized signal, such as those produced by an ADC. The processed result might be a frequency spectrum or a set of statistics. But often it is another quantized signal that is converted back to analog form by a digital-to-analog converter (DAC).

**Domains** - DSP engineers usually study digital signals in one of the following domains: time domain (one-dimensional signals), spatial domain (multidimensional signals), frequency domain, and wavelet domains. They choose the domain in which to process a signal by making an informed assumption (or by trying different possibilities) as to which domain best represents the essential characteristics of the signal and the processing to be applied to it. A sequence of samples from a measuring device produces a temporal or spatial domain representation, whereas a discrete Fourier transform produces the frequency domain representation.

**Time and space domains** -Time domain refers to the analysis of signals with respect to time. Similarly, space domain refers to the analysis of signals with respect to position, e.g., pixel location for the case of image processing.

The most common processing approach in the time or space domain is enhancement of the input signal through a method called filtering. Digital filtering generally consists of some linear transformation of a number of surrounding samples around the current sample of the input or output signal. The surrounding samples may be identified with respect to time or space. The output of a linear digital filter to any given input may be calculated by convolving the input signal with an impulse response.

**Frequency domain** - Signals are converted from time or space domain to the frequency domain usually through use of the Fourier transform. The Fourier transform converts the time or space information to a magnitude and phase component of each frequency. With some applications, how the phase varies with frequency can be a significant consideration. Where phase is unimportant, often the Fourier transform is converted to the power spectrum, which is the magnitude of each frequency component squared.

The most common purpose for analysis of signals in the frequency domain is analysis of signal properties. The engineer can study the spectrum to determine which frequencies are present in the input signal and which are missing. Frequency domain analysis is also called spectrum- or spectral analysis.

Filtering, particularly in non-realtime work can also be achieved in the frequency domain, applying the filter and then converting back to the time domain. This can be an efficient implementation and can give essentially any filter response including excellent approximations to brickwall filters.

There are some commonly used frequency domain transformations. For example, the cepstrum converts a signal to the frequency domain through Fourier transform, takes the logarithm, then applies another Fourier transform. This emphasizes the harmonic structure of the original spectrum.

**Z-plane analysis** - Digital filters come in both infinite impulse response (IIR) and finite impulse response (FIR) types. Whereas FIR filters are always stable, IIR filters have feedback loops that may become unstable and oscillate. The Z-transform provides a tool for analyzing stability issues of digital IIR filters. It is analogous to the Laplace transform, which is used to design and analyze analog IIR filters.

**Autoregression analysis** - A signal is represented as linear combination of its previous samples. Coefficients of the combination are called autoregression coefficients. This method has higher frequency resolution and can process shorter signals compared to the Fourier transform. Prony's method can be used to estimate phases, amplitudes, initial phases and decays of the components of signal. Components are assumed to be complex decaying exponents.

**Time-frequency analysis** -A time-frequency representation of signal can capture both temporal evolution and frequency structure of analyzed signal. Temporal and frequency resolution are limited by the principle of uncertainty and the tradeoff is adjusted by the width of analysis window. Linear techniques such as Short-time Fourier transform, wavelet transform, filter bank, non-linear (e.g., Wigner-Ville transform) and autoregressive methods (e.g. segmented Prony method) are used for representation of signal on the time-frequency plane. Non-linear and segmented Prony methods can provide higher resolution, but may produce undesirable artifacts. Time-frequency analysis is usually used for analysis of non-stationary signals. For example, methods of fundamental frequency estimation, such as RAPT and PEFAC are based on windowed spectral analysis.

**Wavelet** - In numerical analysis and functional analysis, a discrete wavelet transform is any wavelet transform for which the wavelets are discretely sampled. As with other wavelet transforms, a key advantage it has over Fourier transforms is temporal resolution: it captures both frequency and location information. The accuracy of the joint time-frequency resolution is limited by the uncertainty principle of time-frequency.

**Empirical mode decomposition** - Empirical mode decomposition is based on decomposition signal into intrinsic mode functions (IMFs). IMFs are quasiharmonical oscillations that are extracted from the signal.

**Implementation** - DSP algorithms may be run on general-purpose computers and digital signal processors. DSP algorithms are also implemented on purpose-built hardware such as application-specific integrated circuit (ASICs). Additional technologies for digital signal processing include more powerful general purpose microprocessors, graphics processing units, field-programmable gate arrays (FPGAs), digital signal controllers (mostly for industrial applications such as motor control), and stream processors.

For systems that do not have a real-time computing requirement and the signal data (either input or output) exists in data files, processing may be done economically with a general-purpose computer. This is essentially no different from any other data processing, except DSP mathematical techniques (such as the DCT and FFT) are used, and the sampled data is usually assumed to be uniformly sampled in time or space. An example of such an application is processing digital photographs with software such as Photoshop.

When the application requirement is real-time, DSP is often implemented using specialized or dedicated processors or microprocessors, sometimes using multiple processors or multiple processing cores. These may process data using fixed-point arithmetic or floating point. For more demanding applications FPGAs may be used. For the most demanding applications or high-volume products, ASICs might be designed specifically for the application.

Parallel implementations of DSP algorithms, utilising multi-core CPU and many-core GPU architectures, are developed to improve the performances in terms of latency of these algorithms.

Native processing is done by the computer's CPU rather than by DSP or outboard processing, which is done by additional third-party DSP chips located on extension cards or external hardware boxes or racks. Many digital audio workstations such as Logic Pro, Cubase, Digital Performer and Pro Tools LE use native processing. Others, such as Pro Tools HD, Universal Audio's UAD-1 and TC Electronic's Powercore use DSP processing.

**Ultrasonic transducers and ultrasonic sensors** - are devices that generate or sense ultrasound energy. They can be divided into three broad categories: transmitters, receivers and transceivers. Transmitters convert electrical signals into ultrasound, receivers convert ultrasound into electrical signals, and transceivers can both transmit and receive ultrasound.

**Applications and performance** - Ultrasound can be used for measuring wind speed and direction (anemometer), tank or channel fluid level, and speed through air or water. For measuring speed or direction, a device uses multiple detectors and calculates the speed from the relative distances to particulates in the air or water. To measure tank or channel liquid level, and also sea level (tide gauge), the sensor measures the distance (ranging) to the surface of the fluid. Further applications include: humidifiers, sonar, medical ultrasonography, burglar alarms and non-destructive testing.

Systems typically use a transducer that generates sound waves in the ultrasonic range, above 18 kHz, by turning electrical energy into sound, then upon receiving the echo turn the sound waves into electrical energy which can be measured and displayed.

This technology, as well, can detect approaching objects and track their positions.

Ultrasound can also be used to make point-to-point distance measurements by transmitting and receiving discrete bursts of ultrasound between transducers. This technique is known as Sonomicrometry where the transit-time of the ultrasound signal is measured electronically (ie digitally) and converted mathematically to the distance between transducers assuming the speed of sound of the medium between the transducers is known. This method can be very precise in terms of temporal and spatial resolution because the time-of-flight measurement can be derived from tracking the same incident (received) waveform either by reference level or zero crossing. This enables the measurement resolution to far exceed the wavelength of the sound frequency generated by the transducers.

**Ultrasonic transducers** - convert alternating current (AC) into ultrasound and vice versa. The transducers typically use piezoelectric transducers or capacitive transducers to generate or receive ultrasound. Piezoelectric crystals are able to change their sizes and shapes in response to voltage being applied. On the other hand, capacitive transducers use electrostatic fields between a conductive diaphragm and a backing plate.

The beam pattern of a transducer can be determined by the active transducer area and shape, the ultrasound wavelength, and the sound velocity of the propagation medium. The diagrams show the sound fields of an unfocused and a focusing ultrasonic transducer in water, plainly at differing energy levels.

Since piezoelectric materials generate a voltage when force is applied to them, they can also work as ultrasonic detectors. Some systems use separate transmitters and receivers, while others combine both functions into a single piezoelectric transceiver.

Ultrasound transmitters can also use non-piezoelectric principles. such as magnetostriction. Materials with this property change size slightly when exposed to a magnetic field and make practical transducers.

A capacitor ("condenser") microphone has a thin diaphragm that responds to ultrasound waves. Changes in the electric field between the diaphragm and a closely spaced backing plate convert sound signals to electric currents, which can be amplified.

The diaphragm (or membrane) principle is also used in the relatively new micro-machined ultrasonic transducers (MUTs). These devices are fabricated using silicon micro-machining technology (MEMS technology), which is particularly useful for the fabrication of transducer arrays. The vibration of the diaphragm may be measured or induced electronically using the capacitance between the diaphragm and a closely spaced backing plate (CMUT), or by adding a thin layer of piezo-electric material on the diaphragm (PMUT). Alternatively, recent research showed that the vibration of the diaphragm may be measured by a tiny optical ring resonator integrated inside the diaphragm (OMUS).

Ultrasonic Transducers are also used in acoustic levitation.

**Fourier transform (FT)** is a transform that converts a function into a form that describes the frequencies present in the original function. The output of the transform is a complex-valued function of frequency. The term Fourier transform refers to both this complex-valued function and the mathematical operation. When a distinction needs to be made the Fourier transform is sometimes called the frequency domain representation of the original function. The Fourier transform is analogous to decomposing the sound of a musical chord into terms of the intensity of its constituent pitches.

Functions that are localized in the time domain have Fourier transforms that are spread out across the frequency domain and vice versa, a phenomenon known as the uncertainty principle. The critical case for this principle is the Gaussian function, of substantial importance in probability theory and statistics as well as in the study of physical phenomena exhibiting normal distribution (e.g., diffusion). The Fourier transform of a Gaussian function is another Gaussian function. Joseph Fourier introduced the transform in his study of heat transfer, where Gaussian functions appear as solutions of the heat equation.

The Fourier transform can be formally defined as an improper Riemann integral, making it an integral transform, although this definition is not suitable for many applications requiring a more sophisticated integration theory. For example, many relatively simple applications use the Dirac delta function, which can be treated formally as if it were a function, but the justification requires a mathematically more sophisticated viewpoint.

The Fourier transform can also be generalized to functions of several variables on Euclidean space, sending a function of 3-dimensional 'position space' to a function of 3-dimensional momentum (or a function of space and time to a function of 4-momentum). This idea makes the spatial Fourier transform very natural in the study of waves, as well as in quantum mechanics, where it is important to be able to represent wave solutions as functions of either position or momentum and sometimes both. In general, functions to which Fourier methods are applicable are complex-valued, and possibly vector-valued. Still further generalization is possible to functions on groups, which, besides the original Fourier transform on R or Rn, notably includes the discrete-time Fourier transform (DTFT, group = Z), the discrete Fourier transform (DFT, group = Z mod N) and the Fourier series or circular Fourier transform (group = S1, the unit circle ≈ closed finite interval with endpoints identified). The latter is routinely employed to handle periodic functions. The fast Fourier transform (FFT) is an algorithm for computing the DFT.

**Software-defined radio (SDR)** - is a radio communication system where components that conventionally have been implemented in analog hardware (e.g. mixers, filters, amplifiers, modulators/demodulators, detectors, etc.) are instead implemented by means of software on a personal computer or embedded system. While the concept of SDR is not new, the rapidly evolving capabilities of digital electronics render practical many processes which were once only theoretically possible.

A basic SDR system may consist of a personal computer equipped with a sound card, or other analog-to-digital converter, preceded by some form of RF front end. Significant amounts of signal processing are handed over to the general-purpose processor, rather than being done in special-purpose hardware (electronic circuits). Such a design produces a radio which can receive and transmit widely different radio protocols (sometimes referred to as waveforms) based solely on the software used.

Software radios have significant utility for the military and cell phone services, both of which must serve a wide variety of changing radio protocols in real time. In the long term, software-defined radios are expected by proponents like the Wireless Innovation Forum to become the dominant technology in radio communications. SDRs, along with software defined antennas are the enablers of cognitive radio.

Superheterodyne receivers use a VFO (variable-frequency oscillator), mixer, and filter to tune the desired signal to a common IF (intermediate frequency) or baseband. Typically in SDR, this signal is then sampled by the analog-to-digital converter. However, in some applications it is not necessary to tune the signal to an intermediate frequency and the radio frequency signal is directly sampled by the analog-to-digital converter (after amplification).

Real analog-to-digital converters lack the dynamic range to pick up sub-microvolt, nanowatt-power radio signals produced by an antenna. Therefore, a low-noise amplifier must precede the conversion step and this device introduces its own problems. For example, if spurious signals are present (which is typical), these compete with the desired signals within the amplifier's dynamic range. They may introduce distortion in the desired signals, or may block them completely. The standard solution is to put band-pass filters between the antenna and the amplifier, but these reduce the radio's flexibility. Real software radios often have two or three analog channel filters with different bandwidths that are switched in and out.

The flexibility of SDR allows for dynamic spectrum usage, alleviating the need to statically assign the scarce spectral resources to a single fixed service.

**Digital oscilloscopes** - While analog devices make use of continually varying voltages, digital devices employ binary numbers which correspond to samples of the voltage. In the case of digital oscilloscopes, an analog-to-digital converter (ADC) is used to change the measured voltages into digital information. Waveforms are taken as a series of samples. The samples are stored, accumulating until enough are taken in order to describe the waveform, which are then reassembled for display. Digital technology allows the information to be displayed with brightness, clarity, and stability. There are, however, limitations as with the performance of any oscilloscope. The highest frequency at which the oscilloscope can operate is determined by the analog bandwidth of the front-end components of the instrument and the sampling rate.

Digital oscilloscopes can be classified into two primary categories: digital storage oscilloscopes and digital sampling oscilloscopes. Newer variants include PC-based oscilloscopes (which attach to a PC for data processing and display) and mixed-signal oscilloscopes (which employ other functions in addition to voltage measurement).

The digital storage oscilloscope, or DSO for short, is now the preferred type for most industrial applications. Instead of storage-type cathode ray tubes, DSOs use digital memory, which can store data as long as required without degradation. A digital storage oscilloscope also allows complex processing of the signal by high-speed digital signal processing circuits.

The vertical input is digitized by an analog-to-digital converter to create a data set that is stored in the memory of a microprocessor. The data set is processed and then sent to the display, which in early DSOs was a cathode ray tube, but today is a LCD flat panel. DSOs with color LCD displays are common. The sampling data set can be stored to internal or removable storage or sent over a LAN or USB for processing or archiving. A screen image can also be saved to internal or removable storage, or sent to a built-in or externally connected printer, without the need for an oscilloscope camera. The oscilloscope's own signal analysis software can extract many useful time-domain features (e.g., rise time, pulse width, amplitude), frequency spectra, histograms and statistics, persistence maps, and a large number of parameters meaningful to engineers in specialized fields such as telecommunications, disk drive analysis and power electronics..

Digital oscilloscopes are limited principally by the performance of the analog input circuitry, the duration of the sample window, and resolution of the sample rate. When not using equivalent-time sampling, the sampling frequency should be higher than the Nyquist rate which is double the frequency of the highest-frequency component of the observed signal, otherwise aliasing occurs.

Advantages over the analog oscilloscope include:
- Brighter and bigger display with color to distinguish multiple traces
- Simple one-shot acquisitions into memory without the issues that come with storage-type CRTs
- Much more versatile triggers
- No hiding of noise in the phosphor gloom as it happens on analog oscilloscopes
- The input signal is not just converted into a line on the screen, it is available as sample data which can be stored or further processed (i.e. through measurements and analysis tools that come with the oscilloscope)
- Averaging across consecutive samples or scans as well as specific HiRes modes which work through oversampling can lead to higher vertical resolution
- Versatile measurement and analysis functions make it easy to gather all relevant signal properties
- Peak detection to find specific events at long timebase settings on digital oscilloscopes with small memory (less relevant as newer oscilloscopes now come with large memories that keep the sample rate sufficiently high even at very long timebase settings)
- Easy pan and zoom
- Remote control via USB, Ethernet or GPIB

A disadvantage of older digital oscilloscopes is the limited waveform update rate (trigger rate) compared to their analog predecessors, which can make it difficult to spot "glitches" or other rare phenomena with digital oscilloscopes, especially older ones that have no persistence mode. However, thanks to improvements in waveform processing, newer digital oscilloscopes can reach trigger rates in excess of 1 million updates/second, which is more than the roughly 600,000 triggers/sec the best analog oscilloscopes were able to do. Newer digital oscilloscopes also come with analog persistence modes, which replicate the afterglow of an analog oscilloscope's phosphor CRT.

**Digital sampling oscilloscopes** - Digital sampling oscilloscopes operate on the same principle as analog sampling oscilloscopes and, like their analog counterparts, are of great use when analyzing high-frequency signals; that is, repetitive signals whose frequencies are higher than the oscilloscope's sampling rate. For measuring repetitive signals, this type once used to offer bandwidth and high-speed timing up to ten times greater than any real-time oscilloscope.

A real-time oscilloscope, which also used to be called a "single-shot" scope, captures an entire waveform on each trigger event. This requires the scope to capture a large number of data points in one continuous record. A sequential equivalent-time sampling oscilloscope, sometimes simply called a "sampling scope," measures the input signal only once per trigger. The next time the scope is triggered, a small delay is added and another sample is taken. Thus a large number of trigger events must occur in order to collect enough samples to build a picture of the waveform. The measurement bandwidth is determined by the frequency response of the sampler which currently can extend beyond 90 GHz.

An alternative to sequential equivalent-time sampling is called random equivalent-time sampling. Samples are synchronised not with trigger events but with the scope's internal sampling clock. This causes them to occur at apparently random times relative to the trigger event. The scope measures the time interval between the trigger and each sample, and uses this to locate the sample correctly on the x-axis. This process continues until enough samples have been collected to build up a picture of the waveform. The advantage of this technique over sequential equivalent-time sampling is that the scope can collect data from before the trigger event as well as after it, in a similar way to the pre-trigger function of most real-time digital storage scopes. Random equivalent-time sampling can be integrated into a standard DSO without requiring special sampling hardware, but has the disadvantage of poorer timing precision than the sequential sampling method.

However, due to the progress in ADC technology which has led to real-time oscilloscopes with bandwidths over 100 GHz, the demand for digital sampling oscilloscopes has been shrinking, as has the need for integrating equivalent time sampling in real-time oscilloscopes.

**Handheld oscilloscopes** - Handheld oscilloscopes are useful for many test and field service applications. Today, a hand-held oscilloscope is usually a real-time oscilloscope, using a monochrome or color LCD display. Typically, a hand-held oscilloscope has one or two analog input channels, but four-input-channel versions are also available. Some instruments combine the functions of a digital multimeter with the oscilloscope. These usually are lightweight and have good accuracy.

**PC-based oscilloscopes-A** PC-based oscilloscope is a type of digital oscilloscope which relies on a standard PC platform for waveform display and instrument control. In general, there are two types of PC-based oscilloscopes 1)Standalone oscilloscopes which contain an internal PC platform (PC mainboard) - common with upper mid-range and high-end oscilloscopes, 2) External oscilloscopes which connect via USB or Ethernet to a separate PC (desktop or laptop).

At the end of the 1990s, Nicolet and HP introduced the first standalone PC-based oscilloscopes, where the "oscilloscope" part consisted of a specialized signal acquisition system, consisting of an electrical interface providing isolation and automatic gain controls, high-speed analog-to-digital converters, sample memory and on-board Digital Signal Processor (DSPs). The PC part ran Microsoft Windows as the operating system with an oscilloscope application on top, which displayed the waveform data and was used to control the instrument.

Since then, the high-end lines of standalone oscilloscopes of all four major oscilloscope manufacturers (HP/Agilent/Keysight, LeCroy, Tektronix, Rohde & Schwarz) have been based on a PC platform.

The other group of PC-based oscilloscopes are the external oscilloscopes, i.e. where the acquisition system is physically separate from the PC platform. Depending on the exact hardware configuration of the external oscilloscope, the hardware also could be described as a digitizer, a data logger or as a part of a specialized automatic control system. The separate PC provides the display, control interface, disc storage, networking and often the electrical power for the acquisition hardware. The external oscilloscope can transfer data to the computer in two main ways - streaming and block mode. In streaming mode the data is transferred to the PC in a continuous flow without any loss of data. The way in which the PCO is connected to the PC (e.g. Ethernet, USB etc.) will dictate the maximum achievable speed and thereby frequency and resolution using this method. Block mode utilizes the on-board memory of the external oscilloscope to collect a block of data which is then transferred to the PC after the block has been recorded. The acquisition hardware then resets and records another block of data. This process happens very quickly, but the time taken will vary according to the size of the block of data and the speed at which it can be transferred. This method enables a much higher sampling speed, but in many cases the hardware will not record data whilst it is transferring the existing block.

The advantages of a standalone PC-based oscilloscopes include:
- Easy exporting of data to standard PC software such as spreadsheets and word processors which can run on the oscilloscope
- Ability to run analysis tools like numerical analysis software and or signal analysis software directly on the oscilloscope
- Ability to run automation software to perform automatic tests
- Ability to easily control the oscilloscope from a remote location via networking

The advantages of external oscilloscopes are the same as for standalone PC-based oscilloscopes, plus in addition:
- Costs are often lower than for a comparable stand-alone oscilloscope, especially if the user already owns a suitable PC or laptop
- Standalone PCs and laptops typically have large high-resolution color displays which can be easier to read than the smaller displays found on conventional oscilloscopes.
- Portability when used with a laptop PC
- Some external oscilloscopes are much smaller physically than even handheld oscilloscopes

However, PC-based oscilloscopes, standalone or external, also have some disadvantages, which include:
- Power-supply and electromagnetic noise from PC circuits, which requires careful and extensive shielding to obtain good low-level signal resolution
- For external oscilloscopes, the need for the owner to install oscilloscope software on the PC, which may not be compatible with the current release of the PC operating system
- Time for the PC platform to boot, compared with the almost instant start-up of a standalone oscilloscope based on an embedded platform (although every oscilloscope will require a warm-up period to reach specification compliance so this should rarely be an issue)

**Mixed-signal oscilloscopes** -A mixed-signal oscilloscope (MSO) combines all the measurement capabilities and the use model of a Digital Storage Oscilloscope with some of the measurement capabilities of a logic analyzer. Analog and digital signals are acquired with a single time base, they are viewed on a single display, and any combination of these signals can be used to trigger the oscilloscope.

MSOs typically lack the advanced digital measurement capabilities and the large number of digital acquisition channels of standalone logic analyzers. [5] Typical mixed-signal measurement uses include the characterization and debugging of hybrid analog/digital circuits like for example embedded systems, Analog-to-digital converters (ADCs), Digital-to-analog converters (DACs), and control systems.

**Cathode-ray oscilloscope** - The earliest and simplest type of oscilloscope consisted of a cathode ray tube, a vertical amplifier, a timebase, a horizontal amplifier and a power supply. These are now called "analog" oscilloscopes to distinguish them from the "digital" oscilloscopes that became common in the 1990s and 2000s.

A **digital storage oscilloscope (DSO)** is an oscilloscope which stores and analyzes the input signal digitally rather than using analog techniques. It is now the most common type of oscilloscope in use because of the advanced trigger, storage, display and measurement features which it typically provides.

The input analogue signal is sampled and then converted into a digital record of the amplitude of the signal at each sample time. The sampling frequency should be not less than the Nyquist rate to avoid aliasing. These digital values are then turned back into an analogue signal for display on a cathode ray tube (CRT), or transformed as needed for the various possible types of output-liquid crystal display, chart recorder, plotter or network interface.

### Business Model

Currently, prior to Applicant's claimed invention, customers spend hundreds of hours in evaluating a single sample for microplastics, which is a significant barrier to learning about microplastics. At the same time, new regulations are being established which require customers to measure and comply with microplastics thresholds and the current measurement approaches are not fast or efficient enough. As such, AOS' value proposition is to offer a real-time in situ microplastics detection, according to example embodiments, for an affordable price.

Our key partner will be the Southern California Coastal Water Research Project (SCCWRP), which is currently developing guidelines for water treatment facilities on how to implement California's new water treatment regulations. SCCWRP is a key advisor on technologies for water treatment plants and in Phase II they have offered to test AOS' sensor, according to example embodiments. SCCWRP has 14 member agencies; eight of these agencies manage wastewater treatment and management and the remaining six are regulatory agencies. SCCWRP's support may be critical to early adoption of the sensor in water treatment plants in California. AOS has identified both manufacturing and distribution partners during the Phase II project, according to example embodiments.

Because AOS is committed to producing a low-cost sensor, according to example embodiments, we believe it can be widely adopted across a broad range of customer segments. Currently, however, our target customer segments are water treatment plants needing to measure microplastics. AOS plans to use a two-pronged approach to reach customers, according to example embodiments. To reach customers in the water treatment plants, AOS is targeting SCCWRP to be an early adopter of the technology, according to example embodiments, and can rely on using its influence with its member agencies. SCCWRP can also be able to influence other water treatment R&D agencies throughout California. Additionally, AOS can showcase the sensor, according to example embodiments, at trade shows, conferences and direct sales meetings with water treatment plants and water monitoring authorities. To reach additional customers outside of treatment plants, AOS can set up a website and drive customers to the site through search engine optimization. AOS can also leverage its social media channels on Instagram, Facebook, Twitter, and LinkedIn.

AOS can generate revenue through sales of the sensor, according to example embodiments. We plan to use a cost-plus pricing approach to keep the sensor affordable. The key costs can be manufacturing, customer service, and ongoing research and development, since this is an emerging field where rapid iterations are expected.

There are two critical milestones which AOS can meet to address this market opportunity. First, AOS can be successful at creating its own transducers, according to example embodiments, since that is the key cost input in keeping costs low. AOS can decide whether low-cost transducers are successful, according to example embodiments. If AOS needs to use ready-made sensors, the product can still be viable but may cost more. AOS may have a product ready for sales by the end of 2025, according to example embodiments. The CA State Water Resources Control Board has a two-stage testing plan. The first begins in roughly Fall 2023 for two years. After this stage, the State Water Board can analyze the data it receives as well as any new developments in microplastics testing to inform its approach for the second stage, scheduled to begin in Fall 2026. If AOS is not successful in having a product ready for sale by the end of this project, water treatment plants and the state of California can likely identify alternate means to comply with the law.

### The Broad and Niche Market

The first broad market can include all water treatment systems in the United States, including drinking water, wastewater, stormwater, and desalination plants, with the first niche market being drinking water plants in California, according to example embodiments. This is because California is the first in the world to require drinking water agencies to monitor their microplastic load, starting in the autumn of 2023 (California State Water Resources Control Board 2022). We are starting with the domestic market, according to example embodiments, however there is a global market for all of these systems, and desalination, according to example embodiments, in particular is more popular globally than domestically.

Our market can broaden from here with other use cases, such as the 40+ universities, dozens of government labs, and 500+ non-governmental organizations actively monitoring for plastic pollution, as well as the long-term monitoring groups and platforms like ARGO floats, Integrated Ocean Observing System (IOOS), California Cooperative Oceanic Fisheries Investigations (CalCOFI), and Long Term Ecological Research (LTER) Network, that have expressed interest in implementing microplastic monitoring if it was quick and in situ. There are also over 400 research and exploration vessels in the United States alone, all of which could add UltraPlasticsSensors, according to example embodiments, to their repositories of scientific instruments. In addition, there are numerous other water quality user groups, such as agriculture, aquariums, aquaculture, and food and beverage manufacturing, all who are gaining more and more interest in how much microplastics are in both their intake and offtake water supplies.

We have letters of support from a research consortium of 14 leading California water quality management agencies (the Southern California Coastal Water Research Project [SCCWRP]); an academic researcher who studies microplastics (Tammy Russell at Scripps Institution of Oceanography, UC San Diego); a nonprofit who will utilize the sensor for environmental monitoring, education, and community engagement (The Walter Munk Foundation for the Oceans); an environmental engineering firm who specialize in water treatment and who are interested in monitoring microplastics for their customers (Montrose Environmental); and the Save the Waves Coalition who are interested in using our technology to monitor for microplastics, and potentially even harmful algal blooms for conservation and human health reasons. These letters of support illustrate just some of the broad range of potential user groups for a flow-through microplastics sensor, according to example embodiments.

It is important to note as well that globally, the Intergovernmental Negotiating Committee to Develop an International Legally Binding Instrument on Plastic Pollution, composed of 180 nations, also aims to address the point source releases of microplastics into water bodies, however the final law will not be put into effect until 2025 at the earliest (International Institute of Sustainable Development 2023). Our UltraPlasticsSensor, according to example embodiments, could support these efforts with its inexpensive, near real-time, bulk measurement capabilities. According to our customer discovery interviews, Australia is also in the process of instituting policies requiring the testing of all drinking water for microplastic contamination. We have setup a series of customer discovery interviews within the Australian water treatment community to explore this potential market.

### Size of the Broad Market Drinking Water

On average, in the United States, people use ~82 gallons of drinking water per day. There are over 148,000 active drinking water systems in the US, with just 9% of those systems serving most Americans (over 257 million people), and 91% of these systems serving populations of under 10,000 (2021 Infrastructure Report Card - Drinking Water). We can focus our commercialization efforts on these state-based high-volume systems first, especially those in California. About two-thirds of public spending for capital improvements and investment in water infrastructure has been at the state and local level, rather than the federal level, since the 1980s (2021 Infrastructure Report Card - Drinking Water).

### Wastewater

There are more than 16,000 publicly owned wastewater treatment facilities of various sizes in the United States alone, which serve the majority (approximately 80%) of Americans. The rest of the population have their wastewater treated onsite, with systems such as septic tanks (2021 Infrastructure Report Card - Wastewater). Of those public wastewater systems, 11.2% were noncompliant or had a significant compliance violation in 2020 (EPA Facility Registry Service 2020), which could lead to large microplastic leaks.

### Stormwater

Stormwater is defined as water that travels over impervious surfaces, landscaped, or agricultural land, and then is collected and deposited into a waterbody (e.g., a lake, river, pond, or ocean). As such, stormwater is a strong potential vector of pollution to the environment. Stormwater systems can take many forms - from gray infrastructure like piped systems, detention basins, ditches, canals, and roadway conveyance systems, to green infrastructure like rain gardens, constructed wetlands, permeable pavements, and roadway bioswales or retention ponds (2021 Infrastructure Report Card - Stormwater).

There is not a national database of stormwater systems, but there are an estimated 270 million storm drains, 3.5 million miles of storm sewers, and 2.5 million stormwater treatment systems of various types around the United States (2021 Infrastructure Report Card - Stormwater). There are also approximately 860 combined stormwater/wastewater systems in the country. These systems can experience overflow in heavy rainwater or snowmelt events where large volumes of untreated or undertreated wastewater are released into local waterbodies (EPA NPDES). Polluted runoff affects water quality - almost 600,000 miles of rivers and streams and more than 13 million acres of lakes, reservoirs, and ponds are considered impaired (EPA Summary of State). The UltraPlasticsSensor, according to example embodiments, could be added at multiple monitoring points along these systems.

### Desalination Plants

Desalination provides the ability to access seawater, brackish groundwater, or wastewater effluent that could otherwise be unsuitable for human use or agriculture. Desalination removes salts as well as other contaminants like dissolved metals, pathogens, organic and inorganic matter, and radionuclides (American Membrane Technology Association 2018). In our previous discussions with Poseidon Water, the managers of the Carlsbad Desalination Plant, the largest desalination plant in the Western Hemisphere, it is believed that the reverse osmosis membranes could also remove all microplastics within a certain size range, from drinking water; however, AOS's experimental testing, according to example embodiments, of this water at various locations along the desalination treatment route was permanently postponed due to the pandemic.

There are currently more than 1400 active desalination plants in the US, most treating brackish groundwater. Around the world however, 59% of the approximately 15,900 desalination plants are treating oceanic water, with many arid regions like the Middle East and North Africa relying on this desalted water as their main source of potable water (American Membrane Technology Association 2018; Jones et al. 2019).

### Market Trends

The trends in the water quality market indicate all systems but desalination plants are significantly aging, and governments and managing bodies are thus investing millions of dollars into infrastructure retrofits and improvements. Many are also investing in smart sensor technology to collect real-time data on systems' conditions and to prioritize maintenance schedules. Government agencies like the EPA and the California State Water Resources Control Board are also pushing for stricter water quality standards for other emerging pollutants like PFAS and biologicals. As water management systems retrofit to adapt to these water quality standards, our smart pollutant sensor, according to example embodiments, could be easily incorporated into their existing systems and new standards protocols. We are not adding a huge new machine or cumbersome step to the process but, rather, a small, near real-time sensor, according to example embodiments, to help calculate the abundance of an emerging pollutant and help stakeholders meet the new and upcoming microplastic water quality regulations.

Trends point towards increased investment in desalination infrastructure as there is an increased need for additional freshwater with climate change and urbanization, and current sources unable to meet the demand. Thus, moving towards commercialization partnerships with desalination plants is a logical and practical step.

### Barriers to Entry

One of the big barriers to entry is that there is an estimated annual infrastructure investment gap of $43 billion in the combined U.S. wastewater, stormwater, and drinking water spaces, as these spaces have to update and expand their systems, water quality standards become more stringent, and climate change, extreme weather events, and urbanization exceed the limits of current systems (2021 Infrastructure Report Card-Stormwater). In our customer interviews, it was clear that voluntary monitoring and additional sensors can be unlikely investments at this time, unless there are new regulations passed or significant consumer pressure to know microplastic abundances in the water supply. However, some of this investment gap has recently been met, as the 2021 Bipartisan Infrastructure Bill provided over $50 billion to the EPA for investment into the nation's wastewater, stormwater, and drinking water infrastructure needs, including a combined $9 billion for funds addressing emerging contaminants (EPA Water Infrastructure Investments). The 2022 Inflation Reduction Act will also provide $4 billion to help mitigate drought in the Western United State and $550 million to help disadvantaged communities access potable water (California State Association of Counties; White House Fact Sheet).

Another barrier to entry that arose during interviews is that many companies and industry treatment plants are often only propelled to incorporate changes due to an environmental lawsuit, or due to a threat of an environmental lawsuit. However, when we interviewed a nonprofit that files many of these lawsuits, specifically focusing on organizations that violate wastewater treatment regulations and create point sources of pollution, they cannot currently monitor for microplastic because the current methods are too inefficient and expensive. Based on this information, it is apparent that our UltraPlasticsSensor, according to example embodiments, may be able to better help enforce environmental regulations and move the entire water industry forward by making microplastic measurements easier and quicker.

A final barrier to entry of note is for our intended beachhead market, the sensor would likely require regulatory approval as a testing device. In the case of California drinking water, testing is overseen by the California Division of Drinking Water. Our interviews suggested that approval may be sought at the municipal level in addition to the state level. Furthermore, in California at least, wastewater treatment is a separate system and would likely require a separate approval. Our interviews repeatedly stressed that microplastic monitoring is being mandated with an existing compliance method. As a result, the regulatory environment is not clear. The legislation, as written, specifies two optical sensing devices, but also specifies the need for additional bulk sensors that could signal the need for further testing. Our sensor, according to example embodiments, fits this category and offers an excellent value proposition in the current landscape.

### Current Technologies Available

The current technologies available for microplastic sampling are either imprecise (a visual microscope for >100 mm particles, $700-3000) or extremely expensive (>$25,000-$300,000 for FTIR, Raman spectrometer, or gas chromatography-mass spectrometry), and require extreme precision, multiple manhours per sample for sample collection and analysis, and expert training. All these methods besides a microscope are also very precise, giving particle-by-particle readings, rather than a bulk abundance of plastics in a water sample. There is currently no technology on the market that can give a bulk abundance, in lab or in situ.

### The UltraPlasticsSensor According to Example Embodiments

The technical innovation is an ultrasound-based acoustic sensor, according to example embodiments, that can quickly measure microplastic concentrations in water samples. Such a sensor does not currently exist. The innovative technical advance that makes this possible is our use of the ultrasonic frequency-dependent modalities introduced by microplastic particles and fibers' resonant vibrational modes to quantify concentration and size of plastics in the water sample, according to example embodiments. This allows users to more efficiently process water samples to determine plastic concentrations, thus improving our knowledge of the extent of the global microplastic problem.

Our key value proposition is that we reduce sampling time from hours or days per sample to under ten minutes, or less, per sample, and we do not require extensive sample filtration and preparation, according to example embodiments. Achieving sensing at such short times, i.e., under 10 minutes amounts to real time or near real time, and thus, according to example embodiments, addresses a longfelt and unmet need. Microplastics can be left suspended in water for analysis via the UltraPlasticsSensor, according to example embodiments. The device, according to example embodiments, is also less expensive than many of its conventional counterparts, comes in different tiers of acoustic frequencies for different size classes of plastics, and thus different customer use cases, and provides a bulk abundance of an entire water sample rather than requiring each microplastic particle to be analyzed one at a time. This is essential for many of our potential customers we interviewed who want to test water quality or know bulk plastics concentrations of multiple samples of water without needing to know polymer type, shape, and size, of each sample. The ability to test for bulk microplastics abundance in a sample, according to example embodiments, in under ten minutes is what can allow our solution to win in the marketplace. This ability can fill knowledge gaps and lead to better informed remediation strategies and policy decisions, which can lead to a subsequent reduction of microplastics in the environment, from the ocean to our drinking water.

FIG. 10 depicts an example illustrative embodiment of an example block diagram 1000 of an example computer processor-based example controller as may be used in various example components, according to one example embodiment.

FIG. 10 depicts an exemplary schematic block diagram 1000 illustrating an exemplary computer/communications device hardware architecture as may be used in various exemplary components of an exemplary embodiment including, but not limited to, controller(s), software defined radio device(s), signal generator(s), oscilloscope(s), data acquisition systems, data storage and/or retrieval systems, web servers, application servers, server or client devices, wearable devices, mobile devices, portable devices, personal computer(s), external computer device(s), computing devices, communication devices, embedded device(s), thin or fat client, cloud-based computing devices, content servers, web servers, database servers and/or other application servers, enduser devices, operations center devices, client and/or server devices, network routers, gateways, access points, and the like, according to various exemplary embodiments.

FIG. 10 depicts an exemplary schematic block diagram 1000 illustrating an exemplary computer/communications device hardware architecture as may be used in various exemplary components of an exemplary embodiment including mobile devices, wearables, cloud-based computing devices, content servers, web servers, database servers and/or other application servers, according to various exemplary embodiments. Diagram 1000 may include any of various exemplary computer systems as may be used as an external computer device, an internal and/or STB or SOC, a mobile device, a wearable, a client, or server, web server, application server, and/or any other of the computing devices included in the other drawings, according to various exemplary embodiments. FIG. 10 depicts an exemplary diagram 1000 illustrating an exemplary computer/communications device hardware architecture as may be used in various components of exemplary embodiments of the present invention. FIG. 10 depicts an exemplary view 1000 of an exemplary computer systems such as those represented in other illustrations including, e.g., FIGs. 1B, 2-9, including devices including special purpose devices and/or state machines that may implement certain logic in hardware, and/or using special purpose hardware customized by particular special purpose software instructions,which may execute various modules, including data acquisition, storing, analysis, processing, retrieval, digital capture of analog to digital signal data, encryption of data, security devices, computing devices which may execute various operating system and software components on such computing devices and/or subsystem devices and hardware/software modules as may be represented by various labeled elements or components of the Figures, as may be used in implementing an exemplary embodiment of the present invention. FIG. 10 depicts an exemplary embodiment of a computer system that may be used in computing devices such as, e.g., but not limited to, data acquisition, transducer transmitter and/or receiver, processing, analysis devices of example illustrated test environment, devices, servers, application servers, web servers, client, server, portable, handheld, desktop, floor standing, rack mounted, or other format, or wearable and/or mobile devices, sensor capture devices, routers, gateways, data network communication equipment, according to an exemplary embodiment of the present invention. FIG. 10 depicts an exemplary embodiment of a computer system that may be used as client device, a server device (not shown), a network component, router, packet monitor/analyzer, server device, storage, and/or cloud based storage device, application servers, and/or web servers, etc. (not all shown). The present invention (or any part(s) or function(s) thereof) may be implemented using a hardware state machine, a field programmable gate array (FPGA), an application specific integrated circuit (ASIC), hardware, software, firmware, or a combination thereof and may be implemented in one or more computer systems, controller(s), or other processing systems. In fact, in one exemplary embodiment, the invention may be directed toward one or more computer systems capable of carrying out the functionality described herein. An example of a computer system 1000 is shown in FIG. 10, depicting an exemplary embodiment of a block diagram of an exemplary computer system useful for implementing the present invention. Specifically, FIG. 10 illustrates an example computer 1000, which in an exemplary embodiment may be, e.g., (but not limited to) a personal computer (PC) system running an operating system such as, e.g., (but not limited to) WINDOWS MOBILETM for POCKET PC, or MICROSOFT^{®} WINDOWS^{®} NT/98/2000/XP/CE/7/8/10, etc. available from MICROSOFT^{®} Corporation of Redmond, Wash, U.S.A., SOLARIS^{®} from SUN^{®} Microsystems of Santa Clara, Calif, U.S.A, OS/2 from IBM^{®} Corporation of Armonk, N.Y, U.S.A, Mac/OS, Mac OSX, iOS, from APPLE^{®} Corporation of Cupertino, Calif, U.S.A, etc., or any of various versions of UNIX^{®} (a trademark of the Open Group of San Francisco, Calif, USA) including, e.g., LINUX^{®}, UBUNTU, HPUX^{®}, IBM AIX^{®}, and SCO/UNIX^{®}, etc., ANDROID, Android OS is available from Google, JAVA from Oracle, etc. However, the invention may not be limited to these platforms. Instead, the invention may be implemented on any appropriate computer system running any appropriate operating system. In one exemplary embodiment, the present invention may be implemented on a computer system operating as discussed herein. An exemplary computer system, computer 1000 is shown in FIG. 10. Other components of the invention, such as, e.g., (but not limited to) a computing device, a communications device, a telephone, a personal digital assistant (PDA), a personal computer (PC), a handheld PC, client workstations, thin clients, thick clients, proxy servers, network communication servers, remote access devices, client computers, server computers, routers, web servers, data, media, audio, video, telephony or streaming technology servers, a tablet, a phone, a mobile phone, a cellular phone, a communications device, an iPhone, iOS, a smartphone, an iPad, a tablet based device, an ANDROID OS device, an iOS device, a Symbian based device, a Windows 8, 10, etc., device, etc., may also be implemented using a computer such as that shown in FIG. 10.

The computer system 1000 may include one or more processors, such as, e.g., but not limited to, processor(s) 1004, which may include microprocessors, coprocessors, nanoprocessors, microcontrollers, systems on a chip (SOC), multi-processor systems, parallel processors, CISC type processors, RISC type processors, POWER type processors, ARM-architecture processors, massively parallel processor, graphic processors (GPUs) 1032, cryptographic processors such as, e.g., but not limited to, encryption/decryption processor 1036, quantum computers, etc. The processor(s) 1004 may be connected to a communication infrastructure 1006 (e.g., but not limited to, a communications bus, cross-over bar, or network, etc.). Various exemplary software embodiments may be described in terms of this exemplary computer system. After reading this description, it will become apparent to a person skilled in the relevant art(s) how to implement the invention using other computer systems and/or architectures.

Computer system 1000 may include a display interface 1002 that may forward, e.g., but not limited to, graphics, text, and other data, etc., from the communication infrastructure 1006 (or from a frame buffer, etc., not shown) for display on the display unit 1030, and/or GPU 1032, and/or touchscreen 1034, and/or other input or output, and/or input and output device, sensor based device, low frequency, high frequency, or super high frequency transducers, transmitters, or receivers, signal generators, oscilloscopes, digital oscilloscopes, software defined radio (SDR), etc.

The computer system 1000 may also include, e.g., but may not be limited to, a main memory 1008, random access memory (RAM), and a secondary memory 1010, etc. The secondary memory 1010 may include, for example, (but not limited to) a hard disk drive 1012 and/or a removable storage drive 1014, representing a floppy diskette drive, a magnetic tape drive, an optical disk drive, a compact disk drive CD-ROM, DVD, Personal Cloud storage, redundant array of inexpensive disks (RAID) array, etc. The removable storage drive 1014 may, e.g., but not limited to, read from and/or write to a removable storage unit 1018 in a well-known manner. Removable storage unit 1018, also called a program storage device or a computer program product, may represent, e.g., but not limited to, a floppy disk, magnetic tape, optical disk, compact disk, etc. which may be read from and written to by removable storage drive 1014. As will be appreciated, the removable storage unit 1018 may include a computer usable storage medium having stored therein computer software and/or data.

In alternative exemplary embodiments, secondary memory 1010 may include other similar devices for allowing computer programs or other instructions to be loaded into computer system 1000. Such devices may include, for example, but not limited to, a removable storage unit 1022 and an interface 1020. Examples of such may include a program cartridge and cartridge interface (such as, e.g., but not limited to, those found in video game devices), a removable memory chip (such as, e.g., but not limited to, an erasable programmable read only memory (EPROM), or programmable read only memory (PROM) and associated socket, and other removable storage units 1022 and interfaces 1020, which may allow software and data to be transferred from the removable storage unit 1022 to computer system 1000.

The computing device 1000 may also include a cloud-accessible or cloud-based processing and/or storage solution as may be available from Amazon Web Services available from Amazon of Seattle, WA USA, or Azure cloud available from Microsoft Corporation of Redmond, WA USA, or Google Cloud Service available from Google of Alphabet Corporation, Mountain View, CA USA, among many other network and software communications offerings available from IBM Corporation, Oracle Corporation, and others.

Computer 1000 may also include an input device such as, e.g., (but not limited to) a mouse or other pointing device such as a digitizer, and a keyboard or other data entry device (none of which are labeled).

Computer 1000 may also include output devices, such as, e.g., (but not limited to) display 1030, and display interface 1002. Computer 1000 may include input/output (I/O) devices such as, e.g., (but not limited to) communications interface 1024, cable 1028 and communications path 1026, etc. These devices may include, e.g., but not limited to, a network interface card, and modems (neither are labeled). Communications interface 1024 may allow software and data to be transferred between computer system 1000 and external devices. Examples of communications interface 1024 may include, e.g., but may not be limited to, a modem, a network interface (such as, e.g., an Ethernet card), a communications port, a Personal Computer Memory Card International Association (PCMCIA) slot and card, etc. Software and data transferred via communications interface 1024 may be in the form of signals 1028 which may be electronic, electromagnetic, optical or other signals capable of being received by communications interface 1024. These signals 1028 may be provided to communications interface 1024 via, e.g., but not limited to, a communications path 1026 (e.g., but not limited to, a channel). This channel 1026 may carry signals 1028, which may include, e.g., but not limited to, propagated signals, and may be implemented using, e.g., but not limited to, wire or cable, fiber optics, a telephone line, a cellular link, a radio frequency (RF) link and other communications channels, etc.

In this document, the terms "computer program medium" and "computer readable medium" may be used to generally refer to media such as, e.g., but not limited to removable storage drive 1014, a hard disk installed in hard disk drive 1012, and signals 1028, etc. These computer program products may provide software to computer system 1000. The invention may be directed to such computer program products.

References to "one embodiment," "an embodiment," "example embodiment," "various embodiments," etc., may indicate that the embodiment(s) of the invention so described may include a particular feature, structure, or characteristic, but not every embodiment necessarily includes the particular feature, structure, or characteristic. Further, repeated use of the phrase "in one embodiment," or "in an exemplary embodiment," do not necessarily refer to the same embodiment, although they may.

In the following description and claims, the terms "coupled" and "connected," along with their derivatives, may be used. It should be understood that these terms are not intended as synonyms for each other. Rather, in particular embodiments, "connected" may be used to indicate that two or more elements are in direct or indirect physical or electrical contact with each other. "Coupled" may mean that two or more elements are in direct physical or electrical contact. However, "coupled" may also mean that two or more elements are not in direct contact with each other, but yet still co-operate or interact with each other.

An algorithm is here, and generally, considered to be a self-consistent sequence of acts or operations leading to a desired result. These include physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers or the like. It should be understood, however, that all of these and similar terms are to be associated with the appropriate physical quantities and are merely convenient labels applied to these quantities.

Unless specifically stated otherwise, as apparent from the following discussions, it is appreciated that throughout the specification discussions utilizing terms such as "processing," "computing," "calculating," "determining," or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

In a similar manner, the term "processor" may refer to any device or portion of a device that processes electronic data from registers and/or memory to transform that electronic data into other electronic data that may be stored in registers and/or memory. A "computing platform" may comprise one or more processors.

Embodiments of the present invention may include apparatuses for performing the operations herein. An apparatus may be specially constructed for the desired purposes, or it may comprise a general purpose device modified to become a special purpose device, as set forth herein to perform the processing as described to be selectively activated or reconfigured by a software program stored in the device to become a special purpose device capable of performing the subsystem's or submodule's performance functionality and computer and communications systems instructions, and/or by hardware processing such as, e.g., but not limited to, performing certain trusted platform system processing, including exemplary key based encryption/decryption, network monitoring, packet inspection and the like, according to exemplary embodiments.

Embodiments of the invention may be implemented in one or a combination of hardware, firmware, and software. Embodiments of the invention may also be implemented as instructions stored on a machine-readable medium, which may be read and executed by a computing platform to perform the operations described herein. A machine-readable medium may include any mechanism for storing or transmitting information in a form readable by a machine (e.g., a computer). For example, a machine-readable medium may include read only memory (ROM); random access memory (RAM); magnetic disk storage media; optical storage media; flash memory devices; electrical, optical, acoustical or other form of propagated signals (e.g., carrier waves, infrared signals, digital signals, etc.) when nontransitory, and others.

Computer programs (also called computer control logic), may include object oriented computer programs, and may be stored in main memory 1008 and/or the secondary memory 1010 and/or removable storage units 1014, also called computer program products. Such computer programs, when executed, may enable the computer system 1000 to perform the features of the present invention as discussed herein. In particular, the computer programs, when executed, may enable the processor 1004 to provide a method to resolve conflicts during data synchronization according to an exemplary embodiment of the present invention. Accordingly, such computer programs may represent controllers of the computer system 1000.

Various artificial intelligence based analysis techniques may be used herein including neural networks, machine learning, any of various well-known AI and ML techniques and processes (e.g., reinforcement learning, dynamic programming, state action reward state action (SARSA), q learning, supervised learning, unsupervised learning, tensorflow and openAI libraries, cloud computing services, specific APIs, Microsoft cognitive services, Google cloud AI, Watson AI, OpenAI, offerings from Amazon, Facebook, Baidu, Apple, Google, Microsoft, and others, etc. ), and output of such algorithms may be analyzed further as set forth herein to obtain feature vectors and/or to generate AI generative content such as natural language descriptions of data such as produced by large language model (LLM) and/or neural network, convolutional neural network (CNN), and/or other expert system or AI/ML, processing technique, and other data which may be used to provide further guidance to users via decision support systems, executive information systems, and other graphical user interface enabled network and cyber security monitoring and threat analysis management and processing.

In another exemplary embodiment, the invention may be directed to a computer program product comprising a computer readable medium having control logic (computer software) stored therein. The control logic, when executed by the processor 1004, may cause the processor 1004 to perform the functions of the invention as described herein. In another exemplary embodiment where the invention may be implemented using software, the software may be stored in a computer program product and loaded into computer system 1000 using, e.g., but not limited to, removable storage drive 1014, hard drive 1012 or communications interface 1024, etc. The control logic (software), when executed by the processor 1004, may cause the processor 1004 to perform the functions of the invention as described herein. The computer software may run as a standalone software application program running atop an operating system, or may be integrated into the operating system.

In yet another embodiment, the invention may be implemented primarily in hardware using, for example, but not limited to, hardware components such as application specific integrated circuits (ASICs), or one or more state machines, etc. Implementation of the hardware state machine so as to perform the functions described herein will be apparent to persons skilled in the relevant art(s).

In another exemplary embodiment, the invention may be implemented primarily in firmware.

In yet another exemplary embodiment, the invention may be implemented using a combination of any of, e.g., but not limited to, hardware, firmware, and software, etc.

Exemplary embodiments of the invention may also be implemented as instructions stored on a machine-readable medium, which may be read and executed by a computing platform to perform the operations described herein. A machine-readable medium may include any mechanism for storing or transmitting information in a form readable by a machine (e.g., a computer). For example, a machine-readable medium may include read only memory (ROM); random access memory (RAM); magnetic disk storage media; optical storage media; flash memory devices; electrical, optical, acoustical or other form of propagated signals (e.g., carrier waves, infrared signals, digital signals, etc.), and others.

According to an exemplary embodiment, the application system can include an electronic decision support system (DSS) (not shown), policy-based trust platform systems, which can interact, e.g., but not limited to, with a controller, a computer database management system (DBMS), and/or electronic interactive, graphical user interface (GUI) system, etc. Each of the exemplary DSS, DBMS and/or EIGUI system, can then, using e.g., but not limited to, a cryptographic processor and/or a crypto chip controller processor 1036, or the like, can then encrypt the data using electronic encryptor, which can make use of one or more cryptographic algorithm electronic logic, which can include encryption code, a cryptographic combiner, etc., and may be stored in encrypted form, according to an exemplary embodiment, in a computer database storage facility, from computer database storage device, and from there the process can continue with use of the cryptographic algorithm electronic logic, and electronic decryptor, which can decrypt and/or provide a process for decrypting encrypted data, and/or by providing such data to the DSS, the DBMS, or the EIGUI, if authorized. By using encryption/decryption, certain algorithms can be used, as described herein, including, e.g., but not limited to, checksum, AES encryption, RSA, PKI, TLS, FTPS, SFTP, etc. and/or other cryptographic algorithms and/or protocols, according to exemplary embodiments.

### Conclusion

While various embodiments of the present invention have been described above, it should be understood that they have been presented by way of example only, and not limitation. Thus, the breadth and scope of the present invention should not be limited by any of the above-described exemplary embodiments, but should instead be defined only in accordance with the following claims and their equivalents.

Further aspects and advantages of the invention may be appreciated from the following numbered clauses:
1. An apparatus configured to detect small particles of a substance suspended in a matrix using ultrasound, comprising:
   a container device configured to receive a sample in an inner volume, said container device comprising:
   at least one first ultrasonic transducer mechanically coupled to or disposed on at least one first exterior portion of said container device and oriented toward said inner volume of said container device, said at least one said first ultrasonic transducer comprising:
      at least one first ultrasonic transmitter; and
      at least one first ultrasonic receiver;
   at least one of:
      at least one software defined radio (SDR), or
      at least one signal generator and at least one oscilloscope,
      wherein said at least one software defined radio (SDR) or said at least one signal generator and said at least one oscilloscope are electronically coupled to said at least one first ultrasonic transmitter and electronically coupled to said at least one first ultrasonic receiver; and
   at least one electronic controller coupled to said at least one software defined radio (SDR) or said at least one signal generator and said at least one oscilloscope.
2. The apparatus according to clause 1, wherein said container device further comprises:
   at least one second ultrasonic transducer on at least one second exterior portion of said container device, oriented toward said inner volume of said container device.
3. The apparatus according to clause 2, wherein said at least one second ultrasonic transducer is oriented across from said at least one first ultrasonic transducer.
4. The apparatus of clause 2, wherein said at least one second ultrasonic transducer is oriented at an acute angle from a transmission direction of said at least one first ultrasonic transducer.
5. The apparatus according to any one of clauses 1 to 4, wherein said container device comprises at least one or more of:
   wherein said at least one first exterior portion and said at least one second exterior portion comprise portions of a continuous outer surface of said container device at least partially surrounding said inner volume;
   wherein said at least one first exterior portion and said at least one second exterior portion comprise a plurality of portions of an outer surface of said container device;
   wherein said at least one first exterior portion and said at least one second exterior portion comprise a plurality of portions of at least one outer wall of said container device;
   wherein said at least one first exterior portion and said at least one second exterior portion comprise a plurality of portions of at least one cylindrical portion of said container device;
   wherein said at least one first exterior portion and said at least one second exterior portion comprise a plurality of portions of at least one polygonal portion of said container device;
   wherein said at least one first exterior portion and said at least one second exterior portion comprise a plurality of semicircular portions of at least one circular portion of said container device;
   wherein said at least one first exterior portion and said at least one second exterior portion comprise a plurality of wall portions of said container device about an exterior of said inner volume of said container device;
   wherein said container device comprises a shape comprising at least one or more of:
      at least one spherical container;
      at least one polygon shaped container;
      at least one cylindrical container;
      at least one circular cylindrical shaped container;
      at least one circular cross-section shaped container; or
      at least one polygon cross-section shaped container; or
   wherein said container device comprises at least one or more of:
      at least one inlet;
      at least one outlet;
      at least one cavity for receiving the sample;
      at least one internal volume for receiving the sample; or
      at least one vacuum seal.
6. The apparatus according to any one of clauses 2 to 4, further comprising at least one or more of:
   wherein said at least one said second ultrasonic transducer is oriented in a direction opposite of said at least one first exterior portion;
   wherein said at least one said second ultrasonic transducer is oriented in a direction opposite of said at least one said first transducer;
   wherein said at least one said second ultrasonic transducer is oriented at an angle relative to a transmission direction of said at least one said first transducer;
   wherein said at least one said second ultrasonic transducer is oriented at an angle of about orthogonal relative to a transmission direction of said at least one said first transducer;
   wherein said at least one said second ultrasonic transducer is oriented at an angle of about 90 degrees (°) relative to a transmission direction of said at least one said first transducer; or
   wherein said at least one said second ultrasonic transducer is oriented at an angle of about 45 degrees (°) relative to a transmission direction of said at least one said first transducer.
7. The apparatus according to any one of clauses 1 to 6, further comprising at least one or more of:
   at least four ultrasonic transducers;
   at least one oscilloscope coupled to said at least one first transducer;
   at least one oscilloscope to detect and graph at least one signal from said at least one first transducer;
   at least one stir mechanism; or
   at least one sonication mechanism.
8. The apparatus according to clause 7, comprising said at least one stir mechanism and at least one or more of:
   wherein said at least one stir mechanism comprises at least one magnetic stir rod; or
   wherein said at least one stir mechanism is configured to at least one or more of:
      maintain particles in suspension in the sample;
      stir contents of the sample;
      enable stirring of the sample;
      prevent settling of particles from the sample;
      prevent sediment from forming from the sample; or
      prevent separation of composition of mixture of the sample.
9. The apparatus according to clause 7, comprising said at least one sonication mechanism and at least one or more of:
   wherein said at least one sonication mechanism comprises at least one or more of:
      at least one sonication deck unit; or
      at least one sonicator; or
   wherein said at least one sonication mechanism is configured to at least one or more of:
      break up at least one material in the sample;
      break up at least one biological material in the sample;
      create a standing wave;
      sonicate material in the sample; or
      break up material in the sample.
10. The apparatus according to any one of clauses 1 to 9, wherein the sample comprises at least one or more of:
   water;
   water matrix;
   groundwater;
   groundwater matrix;
   fluid;
   fluid matrix;
   matrix;
   soil;
   soil matrix;
   soil water matrix;
   ocean water;
   lake water;
   river water;
   freshwater;
   drinking water; or
   municipal water.
11. A method of detecting at least one small particle of a substance suspended in a matrix of a sample using ultrasound, comprising:
   (a) transmitting at least one ultrasound signal, by at least one ultrasonic transmitter of at least one ultrasonic transducer, wherein said at least one ultrasonic signal comprises ultrasonic energy being transmitted into the sample containing the small particles, and wherein the ultrasonic energy is recorded in at least one location;
   (b) receiving received ultrasonic energy, by at least one ultrasonic receiver of at least one ultrasonic transducer; and
   (c) empirically mapping said received ultrasonic energy, comprising:
      (i) mapping at least one concentration, and
      (ii) mapping at least one composition of the at least one small particle in the matrix.
12. The method according to clause 11, wherein said mapping of said at least one composition comprises at least one or more of:
   1) mapping at least one size property of the at least one small particle in the matrix;
   2) mapping at least one shape property of the at least one small particle in the matrix; or
   3) mapping at least one material property of the at least one small particle in the matrix.
13. The method according to clause 11 or 12, further comprising at least one or more of:
   d) wherein said at least one ultrasonic transmitter comprises a transmission direction, and wherein said at least one ultrasonic receiver is mounted at an acute angle to the transmission direction of said at least one ultrasonic transmitter, and further comprising:
      detecting backscattered and other ultrasonic energy, by said at least one ultrasonic receiver; wherein said at least one ultrasonic transmitter comprises a transmission direction, and wherein said at least one ultrasonic receiver is mounted across from for opposite to the transmission direction of said at least one ultrasonic transmitter, and further comprising:
      detecting transmission of ultrasonic energy through the sample, by said at least one ultrasonic receiver; or
   e) wherein said at least one ultrasonic transmitter comprises a transmission direction, and wherein a plurality of said at least one ultrasonic receivers are mounted at, at least one or more of:
      a plurality of positions, across from, opposite from, or at an angle to, said transmission direction of said at least one ultrasonic transmitter, and further comprising:
      detecting transmission of ultrasonic energy through the sample, by said plurality of said at least one ultrasonic receivers.
14. The method according to any one of clauses 11 to 13, further comprising at least one or more of:
   d) wherein the at least one small particle of the substance comprises at least one or more of:
      micron size particulates;
      1 micron up to 1.2mm sized particulates;
      sub micron particles;
      nano particles;
      greater than 1micron but smaller than 2mm sized;
      under 20 micron sized particles;
      polyvinylchloride (PVC);
      polystyrene;
      Polyethylene terephthalate or polyethylene terephthalate (PET or PETE);
      other plastics;
      a plastic;
      a perfluoroalkyl, or a polyfluoroalkyl (PFAS) particle;
      a fluoropolymer coating particle;
      a nonstick cookware substance particle;
      a polytetrafluoroethylene (PTFE or TEFLON^{®}-branded) particle;
      a firefighting foam PFAS particle;
      a perfluorooctanoic acid (PFOA) particle;
      a perfluorochemicals (PFCs) particle;
      a perfluorooctane sulfonic acid (PFOS) particle;
      a perfluorinated alkylated substance (PFAS) particle;
      a perfluorononanoic acid (PFNA) particle;
      a perfluorobutane sulfonate (PFBS) particle;
      a perfluorohexane sulfonate (PFHxS) particle;
      hexafluoropropylene oxide dimer acid (HFPO-DA or C3 Dimer Acid or GENX^{®}-branded) particle;
      a packaging foam particle;
      a food packaging PFAS particle;
      a household item PFAS particle;
      a stain-resistant PFAS particle;
      a nonstick cookware PFAS particle;
      an outdoor gear durable water repellent coating PFAS particle;
      a plastic particle;
      a microplastic particle; or
      a particle of plastic of size on the order of 5mm, or smaller, in at least one direction; or
   e) wherein the at least one small particle of the substance comprises at least one or more of:
      microbial contaminants;
      chemical contaminants;
      viruses;
      a biological substance;
      a disseminated intravascular coagulation (DIC);
      a sediment inorganic matter (SIM);
      a sediment pore water using solid-phase micro-extraction and gas chromatography/mass spectrometry in selected ion monitoring mode (SPME/GCMS-SIM);
      a sediment organic matter (SOM);
      polycyclic aromatic hydrocarbon (PAH);
      one or more plankton;
      phytoplankton;
      one or more red tide; or
      microbes.
15. The apparatus of any one of clauses 1 to 10, wherein said at least one SDR or said at least one signal generator is configured to:
   generate the at least one ultrasonic signal.
16. The apparatus according to any one of clauses 1 to 10, wherein said at least one electronic controller comprises at least one or more of:
   a microcontroller;
   a personal computer;
   a system on a chip (SOC);
   an ARM processor;
   a graphics processing unit (GPU);
   an electronic computer processor;
   a multi-core computer processor;
   a tablet or phablet communications device; or
   a portable tablet-based computer device.
17. The apparatus according to any one of clauses 1 to 10, further comprising at least one or more of:
   at least one sensor comprising at least one or more of:
      analog processing;
      digital processing;
      ultrasonic resonant spectroscopy;
      attenuation tomography;
      scatterometry; or
      reflection-based imaging;
   at least one first receiver amplifier electronically coupled to said at least one first ultrasonic receiver;
   at least one first transmitter amplifier electronically coupled to said at least one first ultrasonic transmitter;
   at least one analog to digital converter configured to at least one or more of:
      convert an analog signal to digital signal,
      capture or store digital data in at least one storage device;
   at least one fourier transform system performing processing in frequency domain across all frequencies;
   at least one filter configured to at least one or more of:
      filter an analog signal;
      tuning out noise;
      tuning out sediment; or
      tuning out biological material;
   a pre-amplifier configured to amplify an analog signal;
   a sonicator;
   an indicator of sound energy;
   an integration/summation of particulate levels;
   a sonication deck unit;
   at least one intake valve;
   at least one ingress valve;
   at least one egress valve;
   at least one exit valve; or
   at least one physical chamber for housing a given sample comprising at least one or more of:
      an ensonification chamber;
      a spherical chamber;
      a cylindrical chamber;
      a pipe;
      a polygonal cross section chamber;
      an enclosed chamber;
      a circular cross section chamber; or
   a cylindrical circular chamber; or
   a time synchronization between transmitting from the at least one transmitter, and receiving from the at least one receiver.
18. The apparatus according to any one of clauses 1 to 10, further comprising at least one or more of:
   wherein said ultrasonic energy comprises at least one or more of:
      acoustic energy;
      a pulse;
      a continuous wave tone;
      a sweep;
      a chirp; or
      an M-sequence; or
   wherein said at least one ultrasonic receiver comprises at least one or more of:
      an array of receiver transducers;
      a plurality of receiver transducers;
      a circumferential array of receiver transducers about a periphery of a chamber;
      an array of super high frequency receiver transducers;
      a rail comprising a plurality of receiver transducers;
      a moveable rail comprising an array of receiver transducers;
      a plurality of super high frequency transducers movable in relation relative to proximity to the at least one ultrasonic transmitter;
      an aluminum (AL) rail;
      a rail above a chamber; or
   an array of super high frequency receiver transducers comprising at least one or more of:
      a 20-30 MHz receiver transducer;
      a 30-40 MHz receiver transducer;
      a 40-60 MHz receiver transducer; or
      a 60-80 MHz receiver transducer.
19. The apparatus according to any one of clauses 1 to 10, further comprising:
   a plurality of said at least one receiver at a range of angles to a transmission direction of said at least one ultrasonic transmitter, wherein each of said plurality of said at least one receivers is situated at, at least one or more of:
   at an acute angle from transmission direction;
   at 0 degrees from transmission direction;
   at 90 degrees from transmission direction;
   at 30 degrees from transmission direction;
   at 60 degrees from transmission direction;
   at 45 degrees from transmission direction;
   at 180 degrees from transmission direction;
   at 270 degrees from transmission direction;
   at 360 degrees from transmission direction; or
   at an angle of between 0 and 360 from transmission direction.
20. The method according to any one of clauses 11 to 14, further comprising at least one or more of:
   calibrating based a control sample;
   receiving an analog signal and converting it into a digital signal;
   detecting stuff(particulates) in a matrix;
   digitizing (A/D conversion);
   filtering;
   detecting particulate matter processing for travel;
   analyzing, by spectroscopy;
   resolving;
   focusing or forming a coherent beam of sound;
   acoustic processing;
   processing by ultrasonic resonant spectroscopy;
   processing by attenuation tomography;
   processing by scatterometry;
   processing by reflection-based imaging;
   processing ultrasonic frequencies;
   processing high ultrasonic frequencies;
   processing super high ultrasonic frequencies;
   processing high ultrasonic frequencies of 1-20 MHz frequencies;
   processing super high ultrasonic frequencies of >20MHz and <80MHz;
   processing by a regression;
   processing sample and calibrated controlled sample at all frequencies;
   processing for through transmittance;
   processing for back scattering;
   processing filtering for plastics;
   processing reflection;
   processing for density;
   processing for impedance;
   processing for at least one bulk modulus property - seismo-accoustics (material scientists);
   processing for at least one medical property;
   statistical or heuristics processing;
   processing using artificial intelligence or machine learning processing;
   processing by machine learning techniques;
   processing using neural networks; or
   processing using convolutional neural networks.
21. The apparatus according to any one of clauses 1 to 14, further comprising at least one or more of:
   wherein the detector is configured to detect at least one or more of:
      microplastic particles;
      biological material; or
      suspended sediment; or
   wherein the detector is configured to detect at least one or more of:
      particles in a fluid;
      particles in water;
      particles in stormwater;
      particles in saltwater;
      particles in groundwater;
      particles in drinking water;
      particles in ocean water;
      particles in water to be desalinated;
      contaminants in water;
      contaminants in wastewater;
      contaminants in drinking water;
      suspended sediment in drinking water;
      salt in drinking water;
      plankton in drinking water;
      contaminants in fuel;
      buried objects;
      improvised explosive devices in a surf zone;
      particles in a matrix;
      particles in sediment; or
      particles in a complex structure.

## Claims

1. An apparatus configured to detect small particles of a substance suspended in a matrix using ultrasound, comprising:
a container device configured to receive a sample in an inner volume, said container device comprising:
at least one first ultrasonic transducer mechanically coupled to or disposed on at least one first exterior portion of said container device and oriented toward said inner volume of said container device, said at least one said first ultrasonic transducer comprising:
at least one first ultrasonic transmitter; and
at least one first ultrasonic receiver;
at least one of:
at least one software defined radio (SDR), or
at least one signal generator and at least one oscilloscope,
wherein said at least one software defined radio (SDR) or said at least one signal generator and said at least one oscilloscope are electronically coupled to said at least one first ultrasonic transmitter and
electronically coupled to said at least one first ultrasonic receiver; and
at least one electronic controller coupled to said at least one software defined radio (SDR) or said at least one signal generator and said at least one oscilloscope.

2. The apparatus according to claim 1, comprising at least one or more of:
a) wherein said container device further comprises:
at least one second ultrasonic transducer on at least one second exterior portion of said container device,
oriented toward said inner volume of said container device;
b) wherein said container device comprises at least one or more of:
wherein said at least one first exterior portion and said at least one second exterior portion comprise portions of a continuous outer surface of said container device at least partially surrounding said inner volume;
wherein said at least one first exterior portion and said at least one second exterior portion comprise a plurality of portions of an outer surface of said container device;
wherein said at least one first exterior portion and said at least one second exterior portion comprise a plurality of portions of at least one outer wall of said container device;
wherein said at least one first exterior portion and said at least one second exterior portion comprise a plurality of portions of at least one cylindrical portion of said container device;
wherein said at least one first exterior portion and said at least one second exterior portion comprise a plurality of portions of at least one polygonal portion of said container device;
wherein said at least one first exterior portion and said at least one second exterior portion comprise a plurality of semicircular portions of at least one circular portion of said container device;
wherein said at least one first exterior portion and said at least one second exterior portion comprise a plurality of wall portions of said container device about an exterior of said inner volume of said container device;
wherein said container device comprises a shape comprising at least one or more of:
at least one spherical container;
at least one polygon shaped container;
at least one cylindrical container;
at least one circular cylindrical shaped container;
at least one circular cross-section shaped container; or
at least one polygon cross-section shaped container; or
wherein said container device comprises at least one or more of:
at least one inlet;
at least one outlet;
at least one cavity for receiving the sample;
at least one internal volume for receiving the sample; or
at least one vacuum seal; or
(c) further comprising at least one or more of:
at least four ultrasonic transducers;
at least one oscilloscope coupled to said at least one first transducer;
at least one oscilloscope to detect and graph at least one signal from said at least one first transducer;
at least one stir mechanism; or
at least one sonication mechanism.

3. The apparatus according to claim 2, wherein said (a) comprises at least one or more of:
i. wherein said at least one second ultrasonic transducer is oriented across from said at least one first ultrasonic transducer;
ii. wherein said at least one second ultrasonic transducer is oriented at an acute angle from a transmission direction of said at least one first ultrasonic transducer; or
iii. further comprising at least one or more of:
wherein said at least one said second ultrasonic transducer is oriented in a direction opposite of said at least one first exterior portion;
wherein said at least one said second ultrasonic transducer is oriented in a direction opposite of said at least one said first transducer;
wherein said at least one said second ultrasonic transducer is oriented at an angle relative to a transmission direction of said at least one said first transducer;
wherein said at least one said second ultrasonic transducer is oriented at an angle of about orthogonal relative to a transmission direction of said at least one said first transducer;
wherein said at least one said second ultrasonic transducer is oriented at an angle of about 90 degrees (o) relative to a transmission direction of said at least one said first transducer; or
wherein said at least one said second ultrasonic transducer is oriented at an angle of about 45 degrees (o) relative to a transmission direction of said at least one said first transducer.

4. The apparatus according to claim 2, wherein said (c) comprises at least one or more of:
i. comprising said at least one stir mechanism and at least one or more of:
wherein said at least one stir mechanism comprises at least one magnetic stir rod; or
wherein said at least one stir mechanism is configured to at least one or more of:
maintain particles in suspension in the sample;
stir contents of the sample;
enable stirring of the sample;
prevent settling of particles from the sample;
prevent sediment from forming from the sample; or
prevent separation of composition of mixture of the sample; or
ii. comprising said at least one sonication mechanism and at least one or more of:
wherein said at least one sonication mechanism comprises at least one or more of:
at least one sonication deck unit; or
at least one sonicator; or
wherein said at least one sonication mechanism is configured to at least one or more of:
break up at least one material in the sample;
break up at least one biological material in the sample;
create a standing wave;
sonicate material in the sample; or
break up material in the sample.

5. The apparatus according to any one of claims 1 to 4, wherein the sample comprises at least one or more of:
water;
water matrix;
groundwater;
groundwater matrix;
fluid;
fluid matrix;
matrix;
soil;
soil matrix;
soil water matrix;
ocean water;
lake water;
river water;
freshwater;
drinking water; or
municipal water.

6. The apparatus of any one of claims 1 to 5, further comprising at least one or more of:
(a) wherein said at least one SDR or said at least one signal generator is configured to: generate the at least one ultrasonic signal;
(b) wherein said at least one electronic controller comprises at least one or more of:
a microcontroller;
a personal computer;
a system on a chip (SOC);
an ARM processor;
a graphics processing unit (GPU);
an electronic computer processor;
a multi-core computer processor;
a tablet or phablet communications device; or
a portable tablet-based computer device;
(c) at least one sensor comprising at least one or more of:
analog processing;
digital processing;
ultrasonic resonant spectroscopy;
attenuation tomography;
scatterometry; or
reflection-based imaging;
(d) at least one or more of:
at least one first receiver amplifier electronically coupled to said at least one first ultrasonic receiver;
at least one first transmitter amplifier electronically coupled to said at least one first ultrasonic transmitter;
at least one analog to digital converter configured to at least one or more of:
convert an analog signal to digital signal, or
capture or store digital data in at least one storage device;
at least one fourier transform system performing processing in frequency domain across all frequencies;
at least one filter configured to at least one or more of:
filter an analog signal;
tuning out noise;
tuning out sediment; or
tuning out biological material;
a pre-amplifier configured to amplify an analog signal;
a sonicator;
an indicator of sound energy;
an integration/summation of particulate levels;
a sonication deck unit;
at least one intake valve;
at least one ingress valve;
at least one egress valve;
at least one exit valve;
at least one physical chamber for housing a given sample comprising at least one or more of:
an ensonification chamber;
a spherical chamber;
a cylindrical chamber;
a pipe;
a polygonal cross section chamber;
an enclosed chamber;
a circular cross section chamber; or
a cylindrical circular chamber; or
a time synchronization between transmitting from the at least one transmitter, and receiving from the at least one receiver;
(e) wherein said ultrasonic energy comprises at least one or more of:
acoustic energy;
a pulse;
a continuous wave tone;
a sweep;
a chirp; or
an M-sequence; or
wherein said at least one ultrasonic receiver comprises at least one or more of:
an array of receiver transducers;
a plurality of receiver transducers;
a circumferential array of receiver transducers about a periphery of a chamber;
an array of super high frequency receiver transducers;
a rail comprising a plurality of receiver transducers;
a moveable rail comprising an array of receiver transducers;
a plurality of super high frequency transducers movable in relation relative to proximity to the at least one ultrasonic transmitter;
an aluminum (AL) rail;
a rail above a chamber; or
an array of super high frequency receiver transducers comprising at least one or more of:
a 20-30 MHz receiver transducer;
a 30-40 MHz receiver transducer;
a 40-60 MHz receiver transducer; or
a 60-80 MHz receiver transducer;
(f) a plurality of said at least one receiver at a range of angles to a transmission direction of said at least one ultrasonic transmitter, wherein each of said plurality of said at least one receivers is situated at, at least one or more of:
at an acute angle from transmission direction;
at 0 degrees from transmission direction;
at 90 degrees from transmission direction;
at 30 degrees from transmission direction;
at 60 degrees from transmission direction;
at 45 degrees from transmission direction;
at 180 degrees from transmission direction;
at 270 degrees from transmission direction;
at 360 degrees from transmission direction; or
at an angle of between 0 and 360 from transmission direction;
(g) wherein the detector is configured to detect at least one or more of:
microplastic particles;
biological material; or
suspended sediment; or
(h) wherein the detector is configured to detect at least one or more of:
particles in a fluid;
particles in water;
particles in stormwater;
particles in saltwater;
particles in groundwater;
particles in drinking water;
particles in ocean water;
particles in water to be desalinated;
contaminants in water;
contaminants in wastewater;
contaminants in drinking water;
suspended sediment in drinking water;
salt in drinking water;
plankton in drinking water;
contaminants in fuel;
buried objects;
improvised explosive devices in a surf zone;
particles in a matrix;
particles in sediment; or
particles in a complex structure.

7. A method of detecting at least one small particle of a substance suspended in a matrix of a sample using ultrasound, comprising:
(a) transmitting at least one ultrasound signal, by at least one ultrasonic transmitter of at least one ultrasonic transducer, wherein said at least one ultrasonic signal comprises ultrasonic energy being transmitted into the sample containing the small particles, and wherein the ultrasonic energy is recorded in at least one location;
(b) receiving received ultrasonic energy, by at least one ultrasonic receiver of at least one ultrasonic transducer; and
(c) empirically mapping said received ultrasonic energy, comprising:
(i) mapping at least one concentration, and
(ii) mapping at least one composition of the at least one small particle in the matrix.

8. The method according to claim 7, further comprising at least one or more of:
(d) wherein said mapping of said at least one composition comprises at least one or more of:
1) mapping at least one size property of the at least one small particle in the matrix;
2) mapping at least one shape property of the at least one small particle in the matrix; or
3) mapping at least one material property of the at least one small particle in the matrix;
e) wherein said at least one ultrasonic transmitter comprises a transmission direction, and wherein said at least one ultrasonic receiver is mounted at an acute angle to the transmission direction of said at least one ultrasonic transmitter, and further comprising:
detecting backscattered and other ultrasonic energy, by said at least one ultrasonic receiver; wherein said at least one ultrasonic transmitter comprises a transmission direction, and wherein said at least one ultrasonic receiver is mounted across from for opposite to the transmission direction of said at least one ultrasonic transmitter, and further comprising:
detecting transmission of ultrasonic energy through the sample, by said at least one ultrasonic receiver; or
f) wherein said at least one ultrasonic transmitter comprises a transmission direction, and wherein a plurality of said at least one ultrasonic receivers are mounted at, at least one or more of:
a plurality of positions, across from, opposite from, or at an angle to, said transmission direction of said at least one ultrasonic transmitter, and further comprising:
detecting transmission of ultrasonic energy through the sample, by said plurality of said at least one ultrasonic receivers;
g) wherein the at least one small particle of the substance comprises at least one or more of:
micron size particulates;
1 micron up to 1.2mm sized particulates;
sub micron particles;
nano particles;
greater than 1micron but smaller than 2mm sized;
under 20 micron sized particles;
polyvinylchloride (PVC);
polystyrene;
Polyethylene terephthalate or polyethylene terephthalate (PET or PETE);
other plastics;
a plastic;
a perfluoroalkyl, or a polyfluoroalkyl (PFAS) particle;
a fluoropolymer coating particle;
a nonstick cookware substance particle;
a polytetrafluoroethylene (PTFE or TEFLON^{®}-branded) particle;
a firefighting foam PFAS particle;
a perfluorooctanoic acid (PFOA) particle;
a perfluorochemicals (PFCs) particle;
a perfluorooctane sulfonic acid (PFOS) particle;
a perfluorinated alkylated substance (PFAS) particle;
a perfluorononanoic acid (PFNA) particle;
a perfluorobutane sulfonate (PFBS) particle;
a perfluorohexane sulfonate (PFHxS) particle;
hexafluoropropylene oxide dimer acid (HFPO-DA or C3 Dimer Acid or GENX^{®}-branded) particle;
a packaging foam particle;
a food packaging PFAS particle;
a household item PFAS particle;
a stain-resistant PFAS particle;
a nonstick cookware PFAS particle;
an outdoor gear durable water repellent coating PFAS particle;
a plastic particle;
a microplastic particle; or
a particle of plastic of size on the order of 5mm, or smaller, in at least one direction; or
h) wherein the at least one small particle of the substance comprises at least one or more of:
microbial contaminants;
chemical contaminants;
viruses;
a biological substance;
a disseminated intravascular coagulation (DIC);
a sediment inorganic matter (SIM);
a sediment pore water using solid-phase micro-extraction and gas chromatography/mass spectrometry in selected ion monitoring mode (SPME/GCMS-SIM);
a sediment organic matter (SOM);
polycyclic aromatic hydrocarbon (PAH);
one or more plankton;
phytoplankton;
one or more red tide; or
microbes.

9. The method according to claim 7 or 8, further comprising at least one or more of:
calibrating based a control sample;
receiving an analog signal and converting it into a digital signal;
detecting stuff(particulates) in a matrix;
digitizing (A/D conversion);
filtering;
detecting particulate matter processing for travel;
analyzing, by spectroscopy;
resolving;
focusing or forming a coherent beam of sound;
acoustic processing;
processing by ultrasonic resonant spectroscopy;
processing by attenuation tomography;
processing by scatterometry;
processing by reflection-based imaging;
processing ultrasonic frequencies;
processing high ultrasonic frequencies;
processing super high ultrasonic frequencies;
processing high ultrasonic frequencies of 1-20 MHz frequencies;
processing super high ultrasonic frequencies of >20MHz and <80MHz;
processing by a regression;
processing sample and calibrated controlled sample at all frequencies;
processing for through transmittance;
processing for back scattering;
processing filtering for plastics;
processing reflection;
processing for density;
processing for impedance;
processing for at least one bulk modulus property - seismo-accoustics (material scientists);
processing for at least one medical property;
statistical or heuristics processing;
processing using artificial intelligence or machine learning processing;
processing by machine learning techniques;
processing using neural networks; or
processing using convolutional neural networks.
